# EUROPEAN PATENT APPLICATION

(11) **EP 4 512 808 A1**
(43) Date of publication of application: **26.02.2025**
(21) Application number: 23791951.9
(22) Date of filing: 21.04.2023
(51) Int. Cl.: C07D 487/04, A61K 31/519, A61P 35/00

(54) **CRYSTAL OF 7H-PYRROLO[2,3-D]PYRIMIDINE-4-AMINE DERIVATIVE**

(30) Priority: 22.04.2022 JP 2022071154
(71) Applicant: Taiho Pharmaceutical Co., Ltd., Chiyoda-ku, Tokyo 101-8444 (JP)
(72) Inventor: NAKAMURA, Hiroyuki, Tsukuba-shi, Ibaraki 300-2611 (JP); KOBAYAKAWA, Yu, Tsukuba-shi, Ibaraki 300-2611 (JP); HARA, Shoki, Tokushima-shi, Tokushima 771-0194 (JP)
(74) Representative: Wächtershäuser & Hartz Patentanwaltspartnerschaft mbB
(86) International application number: PCT/JP2023/015916
(87) International publication number: WO 2023/204303

(57) **Abstract**

Provided is a crystal of a compound having EGFR inhibition ability or a salt thereof, said crystal being excellent in one or more characteristics of stability, hygroscopicity and oral absorbability. One aspect of the present invention provides a crystal form of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide the diffraction angle (2θ±0.2°) of which has a peak at a preset angle in the X-ray powder diffraction spectrum.

## Description

### TECHNICAL FIELD

The present invention relates to a crystal of a compound having an inhibitory effect against epidermal growth factor receptor (EGFR), 7H-pyrrolo[2,3-d]pyrimidin-4-amine derivative, a method of crystallization thereof, and the like.

### BACKGROUND ART

EGFR is a receptor-type tyrosine kinase and exerts its physiological functions in normal tissue upon binding to epidermal growth factor (EGF) as a ligand. In the epidermal tissue, EGFR contributes to growth and apoptosis inhibition, etc. (Non-patent Literature 1).

Moreover, EGFR is also a kind of oncogene, and amplification of the EGFR gene and high expression and/or mutation of its protein have been known in various cancer types including head and neck cancer, breast cancer, large bowel cancer, esophageal cancer, pancreatic cancer, lung cancer, ovarian cancer, renal cancer, bladder cancer, skin cancer, brain tumor and so on (Non-patent Literature 2). In particular, the number of deaths caused by lung cancer has reached approximately 1,400,000 per year all over the world, and there has been a demand for the development of effective therapy for non-small cell lung cancer because it accounts for over 80% of lung cancer cases (Non-patent Literature 3).

Currently, several EGFR inhibitors have been reported as antitumor agents, and Patent Literature 1 discloses, as a compound with an excellent inhibitory effect against EGFR, 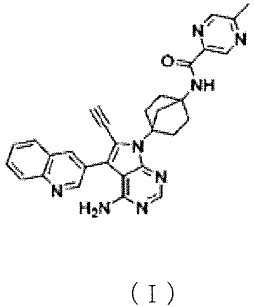 the pyrimidine compound represented by Formula (I) above (chemical name: N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (hereinafter also referred to as "Compound (1)").

Patent Literature 1 discloses a method for producing Compound (1), in which DMF, DIPEA, 5-methylpyrazine-2-carboxylic acid, and HATU are added to a THF solution of 7-(4-aminobicyclo[2.2.1]heptan-1-yl)-6-ethynyl-5-(quinolin-3-yl)-7H-pyrrolo[2.3-d]pyrimidin-4-amine, and the mixture is stirred at room temperature, then concentrated and purified to give the compound. However, Patent Literature 1 does not specifically indicate what kind of crystalline form Compound (1) or a salt thereof can form.

### CITATION LIST

### Patent Literature

Patent Literature 1: WO2020/166680

### Non-patent Literature

Non-patent Literature 1: Nature Rev. Cancer, vol. 6, pp. 803-811 (2006)
Non-patent Literature 2: Current Opinion in Oncology, vol. 13, pp. 506-513 (2001)
Non-patent Literature 3: Lung Cancer, vol. 69, pp. 1-12 (2010)

### SUMMARY OF THE INVENTION

### PROBLEM TO BE SOLVED BY THE INVENTION

When a compound is used as an effective active ingredient in a pharmaceutical product, it is desirable that the compound be in a stable crystalline form in order to maintain stable quality and/or to facilitate storage management. Furthermore, the crystalline form of the compound preferably has low hygroscopicity, good oral absorption, or the like. However, it is difficult to predict whether a particular compound or a salt thereof will form a crystal, and what kind of crystalline form will have excellent physical properties including stability, etc.

Under these circumstances, a crystal of a compound having an inhibitory effect against EGFR or a salt thereof, which is excellent in one or more physical properties including stability, hygroscopicity, and oral absorption, has been desired.

### MEANS TO SOLVE THE PROBLEM

As a result of extensive and intensive studies, the present inventors have obtained several types of crystalline forms of Compound (1) in free form. The present inventors further obtained crystalline forms of Compound (1) with succinic acid, sorbic acid, citric acid, or phosphoric acid as crystalline forms of salts or as co-crystals.

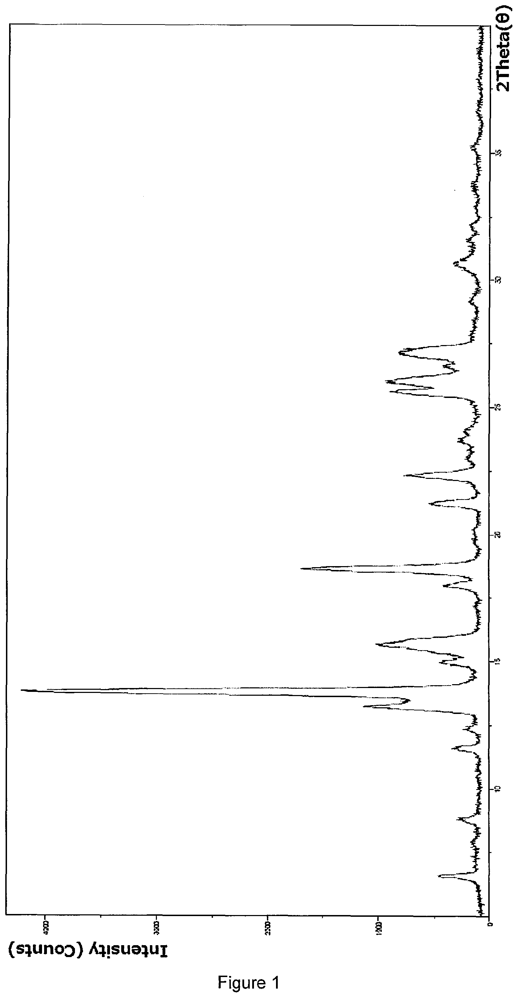

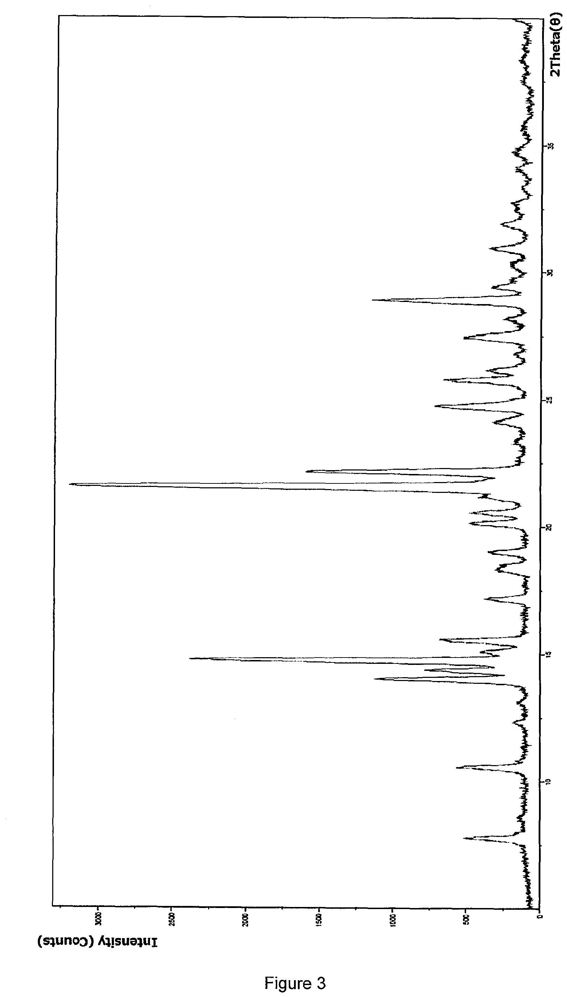

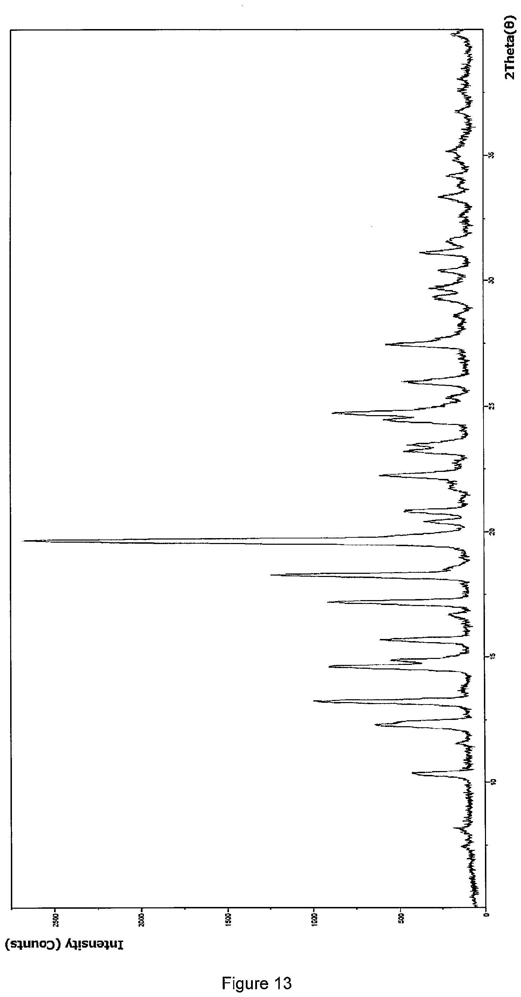

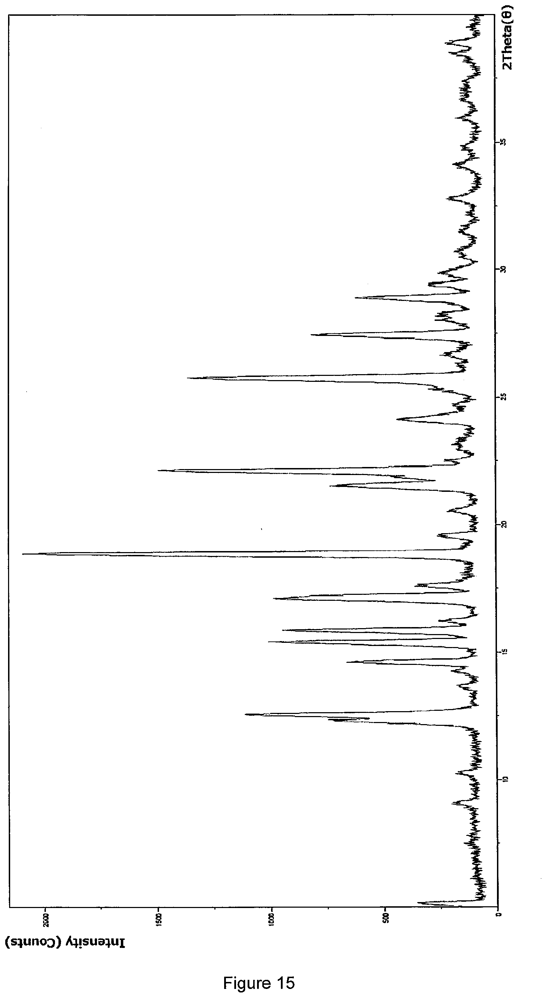

Thus, the present invention provides, for example, [1] to [53] below.
[1] A type II crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide,
   wherein the crystal has peaks in a powder X-ray diffraction spectrum at diffraction angles (2θ ± 0.2°) of:
   (i) one or more selected from the group consisting of 15.6°, 20.1°, 24.7°, and 28.9°; and
   (ii) two or more selected from the group consisting of 7.8°, 10.6°, 14.0°, 14.8°, 17.2°, 21.6°, 22.2°, and 25.8°.
[2] The crystal according to [1] above, wherein the crystal has peaks in a powder X-ray diffraction spectrum at a total of five or more diffraction angles (20 ± 0.2°) selected from groups (i) and (ii) above.
[3] The crystal according to [1] or [2] above, wherein the crystal has peaks in a powder X-ray diffraction spectrum at a total of seven or more diffraction angles (20 ± 0.2°) selected from groups (i) and (ii) above.
[4] The crystal according to any one of [1] to [3] above, wherein the crystal has a powder X-ray diffraction spectrum substantially identical to the powder X-ray diffraction spectrum shown in Figure 3.
[5] The crystal according to any one of [1] to [4] above, wherein an exothermic peak determined by simultaneous thermogravimetric-differential thermal analysis is around 236°C.
[6] The crystal according to any one of [1] to [5] above, wherein the crystal has a crystal purity of 50% by weight or more (preferably 75% by weight or more, more preferably 80% by weight or more, and still more preferably 95% by weight or more).
[7] The crystal according to any one of [1] to [6] above, wherein the crystal has a chemical purity of 90% or more (preferably 95% or more, more preferably 98% or more, and still more preferably 99% or more).
[8] A method for producing the type II crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide according to any one of [1] to [7] above, the method comprising stirring N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide in a single solvent of a lower alcohol with 3 or more carbon atoms, tetrahydrofuran, acetone, or ethyl acetate, or in a mixed solvent comprising two or more of these solvents.
[9] A type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with succinic acid, wherein the crystal has peaks in a powder X-ray diffraction spectrum at three or more diffraction angles (20 ± 0.2°) selected from the group consisting of 10.4°, 13.3°, 14.6°, 15.7°, 17.2°, 18.3°, 19.7°, 20.8°, 22.3°, and 27.5°.
[10] The crystal according to [9] above, wherein the crystal has peaks in a powder X-ray diffraction spectrum at five or more diffraction angles (2θ ± 0.2°) selected from the group consisting of 10.4°, 13.3°, 14.6°, 15.7°, 17.2°, 18.3°, 19.7°, 20.8°, 22.3°, and 27.5°.
[11] The crystal according to [10] or [11] above, wherein the crystal has peaks in a powder X-ray diffraction spectrum at seven or more diffraction angles (20 ± 0.2°) selected from the group consisting of 10.4°, 13.3°, 14.6°, 15.7°, 17.2°, 18.3°, 19.7°, 20.8°, 22.3°, and 27.5°.
[12] The crystal according to any one of [9] to [11] above, wherein the crystal has a powder X-ray diffraction spectrum substantially identical to the powder X-ray diffraction spectrum shown in Figure 13.
[13] The crystal according to any one of [9] to [12] above, wherein an endothermic peak determined by simultaneous thermogravimetric-differential thermal analysis is around 202°C.
[14] The crystal according to any one of [9] to [13] above, wherein the crystal has a crystal purity of 50% by weight or more (preferably 75% by weight or more, more preferably 80% by weight or more, and still more preferably 95% by weight or more).
[15] The crystal according to any one of [9] to [14] above, wherein the crystal has a chemical purity of 90% or more (preferably 95% or more, more preferably 98% or more, and still more preferably 99% or more).
[16] A method for producing the type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with succinic acid according to any one of [9] to [15] above, the method comprising stirring N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide and succinic acid at an equivalent ratio of 1:1-10 in a single solvent selected from acetonitrile, a lower alcohol with 2 or more carbon atoms, methyl ethyl ketone, tetrahydrofuran, or 2-methyltetrahydrofuran, or in a mixed solvent comprising two or more of these solvents.
[17] A type I crystal of N-(4-(4-amino-6-ethynyl-5-(quinolin-3-yl)-7H-pyrrolo[2,3-d]pyrimidin-7-yl)bieyclo[2.2.1]heptan-1-yl)-5-methylpyrazine-2-carboxamide with sorbic acid, wherein the crystal has peaks in a powder X-ray diffraction spectrum at three or more diffraction angles (2θ ± 0.2°) selected from the group consisting of 12.6°, 14.7°, 15.4°, 15.9°, 17.1°, 18.9°, 22.2°, 24.1°, 25.8°, and 27.4°.
[18] The crystal according to [17] above, wherein the crystal has peaks in a powder X-ray diffraction spectrum at five or more diffraction angles (2θ ± 0.2°) selected from the group consisting of 12.6°, 14.7°, 15.4°, 15.9°, 17.1°, 18.9°, 22.2°, 24.1°, 25.8°, and 27.4°.
[19] The crystal according to [17] or [18] above, wherein the crystal has peaks in a powder X-ray diffraction spectrum at seven or more diffraction angles (2θ ± 0.2°) selected from the group consisting of 12.6°, 14.7°, 15.4°, 15.9°, 17.1°, 18.9°, 22.2°, 24.1°, 25.8°, and 27.4°.
[20] The crystal according to any one of [17] to [19] above, wherein the crystal has a powder X-ray diffraction spectrum substantially identical to the powder X-ray diffraction spectrum shown in Figure 15.
[21] The crystal according to any one of [17] to [20] above, wherein an endothermic peak determined by simultaneous thermogravimetric-differential thermal analysis is around 173°C.
[22] The crystal according to any one of [17] to [21] above, wherein the crystal has a crystal purity of 50% by weight or more (preferably 75% by weight or more, more preferably 80% by weight or more, and still more preferably 95% by weight or more).
[23] The crystal according to any one of [17] to [22] above, wherein the crystal has a chemical purity of 90% or more (preferably 95% or more, more preferably 98% or more, and still more preferably 99% or more).
[24] A method for producing the type I crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with sorbic acid according to any one of [17] to [23] above, the method comprising stirring N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide and sorbic acid at an equivalent ratio of 1:1-10 in a single solvent selected from acetonitrile, a lower alcohol, tetrahydrofuran, acetone, methyl ethyl ketone, water, or ethyl acetate, or in a mixed solvent comprising two or more of these solvents.
[25] A type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide, wherein the crystal has peaks in a powder X-ray diffraction spectrum at diffraction angles (2θ ± 0.2°) of:
   (i) one or more selected from the group consisting of 8.8°, 11.6°, and 17.9°; and
   (ii) two or more selected from the group consisting of 6.6°, 12.3°, 13.8°, 18.6°, 21.3°, and 22.3°.
[26] The crystal according to [25] above, wherein the crystal has peaks in a powder X-ray diffraction spectrum at a total of five or more diffraction angles (2θ ± 0.2°) selected from groups (i) and (ii) above.
[27] The crystal according to [25] or [26] above, wherein the crystal has peaks in a powder X-ray diffraction spectrum at a total of seven or more diffraction angles (2θ ± 0.2°) selected from groups (i) and (ii) above.
[28] The crystal according to any one of [25] to [27] above, wherein the crystal has a powder X-ray diffraction spectrum substantially identical to the powder X-ray diffraction spectrum shown in Figure 1.
[29] The crystal according to any one of [25] to [28] above, wherein an exothermic peak determined by simultaneous thermogravimetric-differential thermal analysis is around 228°C.
[30] The crystal according to any one of [25] to [29] above, wherein the crystal has a crystal purity of 50% by weight or more (preferably 75% by weight or more, more preferably 80% by weight or more, and still more preferably 95% by weight or more).
[31] The crystal according to any one of [25] to [30] above, wherein the crystal has a chemical purity of 90% or more (preferably 95% or more, more preferably 98% or more, and still more preferably 99% or more).
[32] A method for producing the type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide according to any one of [25] to [31] above, the method comprising stirring N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide in a methanol-containing solvent.
[33] A pharmaceutical composition comprising the crystal according to any one of [1] to [7], [9] to [15], [17] to [23], and [25] to [31].
[34] A pharmaceutical composition comprising the crystal according to any one of [1] to [7], [9] to [15], [17] to [23], and [25] to [31] and a pharmacologically acceptable carrier.
[35] An antitumor agent comprising the crystal according to any one of [1] to [7], [9] to [15], [17] to [23], and [25] to [31].
[36] A method for treating a tumor, comprising orally administering an effective amount of the crystal according to any one of [1] to [7], [9] to [15], [17] to [23], and [25] to [31] to a subject in need thereof.
[37] Use of the crystal according to any one of [1] to [7], [9] to [15], [17] to [23], and [25] to [31] for the manufacture of an antitumor agent for oral administration.
[38] A type III co-crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with succinic acid, wherein the co-crystal has peaks in a powder X-ray diffraction spectrum at three or more diffraction angles (20 ± 0.2°) selected from the group consisting of 10.4°, 13.3°, 14.6°, 15.7°, 17.2°, 18.3°, 19.7°, 20.8°, 22.3°, and 27.5°.
[39] The co-crystal according to [38] above, wherein the co-crystal has peaks in a powder X-ray diffraction spectrum at five or more diffraction angles (2θ ± 0.2°) selected from the group consisting of 10.4°, 13.3°, 14.6°, 15.7°, 17.2°, 18.3°, 19.7°, 20.8°, 22.3°, and 27.5°.
[40] The co-crystal according to [38] or [39] above, wherein the co-crystal has peaks in a powder X-ray diffraction spectrum at seven or more diffraction angles (2θ ± 0.2°) selected from the group consisting of 10.4°, 13.3°, 14.6°, 15.7°, 17.2°, 18.3°, 19.7°, 20.8°, 22.3°, and 27.5°.
[41] The co-crystal according to any one of [38] to [40] above, wherein the co-crystal has a powder X-ray diffraction spectrum substantially identical to the powder X-ray diffraction spectrum shown in Figure 13.
[42] The co-crystal according to any one of [38] to [41] above, wherein an endothermic peak determined by simultaneous thermogravimetric-differential thermal analysis is around 202°C.
[43] The co-crystal according to any one of [38] to [42] above, wherein the co-crystal has a crystal purity of 50% by weight or more (preferably 75% by weight or more, more preferably 80% by weight or more, and still more preferably 95% by weight or more).
[44] The co-crystal according to any one of [38] to [43] above, wherein the co-crystal has a chemical purity of 90% or more (preferably 95% or more, more preferably 98% or more, and still more preferably 99% or more).
[45] A method for producing the type III co-crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with succinic acid according to any one of [9] to [15] above, the method comprising stirring N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide and succinic acid at an equivalent ratio of 1:1-10 in a single solvent selected from acetonitrile, a lower alcohol with 2 or more carbon atoms, methyl ethyl ketone, tetrahydrofuran, or 2-methyltetrahydrofuran, or in a mixed solvent comprising two or more of these solvents.
[46] A type I co-crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with sorbic acid, wherein the co-crystal has peaks in a powder X-ray diffraction spectrum at three or more diffraction angles (2θ ± 0.2°) selected from the group consisting of 12.6°, 14.7°, 15.4°, 15.9°, 17.1°, 18.9°, 22.2°, 24.1°, 25.8°, and 27.4°.
[47] The co-crystal according to [46] above, wherein the co-crystal has peaks in a powder X-ray diffraction spectrum at five or more diffraction angles (2θ ± 0.2°) selected from the group consisting of 12.6°, 14.7°, 15.4°, 15.9°, 17.1°, 18.9°, 22.2°, 24.1°, 25.8°, and 27.4°.
[48] The co-crystal according to [46] or [47] above, wherein the co-crystal has peaks in a powder X-ray diffraction spectrum at seven or more diffraction angles (2θ ± 0.2°) selected from the group consisting of 12.6°, 14.7°, 15.4°, 15.9°, 17.1°, 18.9°, 22.2°, 24.1°, 25.8°, and 27.4°.
[49] The co-crystal according to any one of [46] to [48] above, wherein the co-crystal has a powder X-ray diffraction spectrum substantially identical to the powder X-ray diffraction spectrum shown in Figure 15.
[50] The co-crystal according to any one of [46] to [49] above, wherein an endothermic peak determined by simultaneous thermogravimetric-differential thermal analysis is around 173°C.
[51] The co-crystal according to any one of [46] to [50] above, wherein the co-crystal has a crystal purity of 50% by weight or more (preferably 75% by weight or more, more preferably 80% by weight or more, and still more preferably 95% by weight or more).
[52] The co-crystal according to any one of [46] to [51] above, wherein the co-crystal has a chemical purity of 90% or more (preferably 95% or more, more preferably 98% or more, and still more preferably 99% or more).
[53] A method for producing the type I co-crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with sorbic acid according to any one of [17] to [23] above, the method comprising stirring N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide and sorbic acid at an equivalent ratio of 1:1-10 in a single solvent selected from acetonitrile, a lower alcohol, tetrahydrofuran, acetone, methyl ethyl ketone, water, or ethyl acetate, or in a mixed solvent comprising two or more of these solvents.

### EFFECTS OF THE INVENTION

According to an embodiment of the present invention, a crystal of a compound having an inhibitory effect against EGFR or a salt thereof, which is excellent in one or more physical properties including stability, hygroscopicity, and oral absorption, is provided.

### BRIEF DESCRIPTION OF DRAWINGS

Figure 1 shows a powder X-ray diffraction spectrum of a type III crystal of Compound (1) in free form obtained in Example 1 (vertical axis represents intensity (counts) and horizontal axis represents diffraction angle (2θ)).
Figure 2 shows results of simultaneous thermogravimetric-differential thermal analysis (TG-DTA) of the type III crystal of Compound (1) in free form obtained in Example 1 (left vertical axis represents weight (%) in the TG curve, right vertical axis represents heat flux (µV) in the DTA curve, and horizontal axis represents temperature (°C)).
Figure 3 shows a powder X-ray diffraction spectrum of a type II crystal of Compound (1) in free form obtained in Example 2 (vertical axis represents intensity (counts) and horizontal axis represents diffraction angle (2θ)).
Figure 4 shows results of simultaneous thermogravimetric-differential thermal analysis (TG-DTA) of the type II crystal of Compound (1) in free form obtained in Example 2 (left vertical axis represents weight (%) in the TG curve, right vertical axis represents heat flux (µV) in the DTA curve, and horizontal axis represents temperature (°C)).
Figure 5 shows a powder X-ray diffraction spectrum of a type VII crystal of Compound (1) in free form obtained in Reference example 1 (vertical axis represents intensity (counts) and horizontal axis represents diffraction angle (2θ)).
Figure 6 shows results of simultaneous thermogravimetric-differential thermal analysis (TG-DTA) of the type VII crystal of Compound (1) in free form obtained in Reference example 1 (left vertical axis represents weight (%) in the TG curve, right vertical axis represents heat flux (µV) in the DTA curve, and horizontal axis represents temperature (°C)).
Figure 7 shows a powder X-ray diffraction spectrum of a type VIII crystal of Compound (1) in free form obtained in Comparative example 1 (vertical axis represents intensity (counts) and horizontal axis represents diffraction angle (2θ)).
Figure 8 shows results of simultaneous thermogravimetric-differential thermal analysis (TG-DTA) of the type VIII crystal of Compound (1) in free form obtained in Comparative example 1 (left vertical axis represents weight (%) in the TG curve, right vertical axis represents heat flux (µV) in the DTA curve, and horizontal axis represents temperature (°C)).
Figure 9 shows results of dynamic vapor sorption (DVS) test for the type II crystal of Compound (1) in free form in Test Example 2.
Figure 10 shows results of dynamic vapor sorption (DVS) test for the type III crystal of Compound (1) in free form in Test Example 2.
Figure 11 shows results of dynamic vapor sorption (DVS) test for the type VII crystal of Compound (1) in free form in Test Example 2.
Figure 12 shows results of dynamic vapor sorption (DVS) test for the type VIII crystal of Compound (1) in free form in Test Example 2.
Figure 13 shows a powder X-ray diffraction spectrum of a type III crystal of Compound (1) with succinic acid obtained in Example 3 (vertical axis represents intensity (counts) and horizontal axis represents diffraction angle (2θ)).
Figure 14 shows results of simultaneous thermogravimetric-differential thermal analysis (TG-DTA) of the type III crystal of Compound (1) with succinic acid obtained in Example 3 (left vertical axis represents weight (%) in the TG curve, right vertical axis represents heat flux (µV) in the DTA curve, and horizontal axis represents temperature (°C)).
Figure 15 shows a powder X-ray diffraction spectrum of a type I crystal of Compound (1) with sorbic acid obtained in Example 4 (vertical axis represents intensity (counts) and horizontal axis represents diffraction angle (2θ)).
Figure 16 shows results of simultaneous thermogravimetric-differential thermal analysis (TG-DTA) of the type I crystal of Compound (1) with sorbic acid obtained in Example 4 (left vertical axis represents weight (%) in the TG curve, right vertical axis represents heat flux (µV) in the DTA curve, and horizontal axis represents temperature (°C)).
Figure 17 shows a powder X-ray diffraction spectrum of a type I crystal of Compound (1) with citric acid obtained in Comparative example 2 (vertical axis represents intensity (counts) and horizontal axis represents diffraction angle (2θ)).
Figure 18 shows results of simultaneous thermogravimetric-differential thermal analysis (TG-DTA) of the type I crystal of Compound (1) with citric acid obtained in Comparative example 2 (left vertical axis represents weight (%) in the TG curve, right vertical axis represents heat flux (µV) in the DTA curve, and horizontal axis represents temperature (°C)).
Figure 19 shows a powder X-ray diffraction spectrum of a type IV crystal of Compound (1) with citric acid obtained in Comparative example 3 (vertical axis represents intensity (counts) and horizontal axis represents diffraction angle (2θ)).
Figure 20 shows results of simultaneous thermogravimetric-differential thermal analysis (TG-DTA) of the type IV crystal of Compound (1) with citric acid obtained in Comparative example 3 (left vertical axis represents weight (%) in the TG curve, right vertical axis represents heat flux (µV) in the DTA curve, and horizontal axis represents temperature (°C)).
Figure 21 shows a powder X-ray diffraction spectrum of a type I crystal of Compound (1) with phosphoric acid obtained in Comparative example 4 (vertical axis represents intensity (counts) and horizontal axis represents diffraction angle (2θ)).
Figure 22 shows results of simultaneous thermogravimetric-differential thermal analysis (TG-DTA) of the type I crystal of Compound (1) with phosphoric acid obtained in Comparative example 4 (left vertical axis represents weight (%) in the TG curve, right vertical axis represents heat flux (µV) in the DTA curve, and horizontal axis represents temperature (°C)).
Figure 23 shows results of dynamic vapor sorption (DVS) test for the type III crystal of Compound (1) with succinic acid in Test Example 4.
Figure 24 shows results of dynamic vapor sorption (DVS) test for the type I crystal of Compound (1) with citric acid in Test Example 4.
Figure 25 shows results of dynamic vapor sorption (DVS) test for the type IV crystal of Compound (1) with citric acid in Test Example 4.
Figure 26 shows results of dynamic vapor sorption (DVS) test for the type I crystal of Compound (1) with sorbic acid in Test Example 4.
Figure 27 shows results of dynamic vapor sorption (DVS) test for the type I crystal of Compound (1) with phosphoric acid in Test Example 4.

### DESCRIPTION OF EMBODIMENTS

The present invention relates to a crystal of N-(4-(4-amino-6-ethynyl-5-(quinolin-3-yl)-7H-pyrrolo[2,3-d]pyrimidin-7-yl)bicyclo[2.2.11]heptan-1-yl)-5-methylpyrazine-2-carboxamide represented by Formula (1) below, and a crystal of said compound with an acid (a crystal of a salt or a co-crystal of said compound).

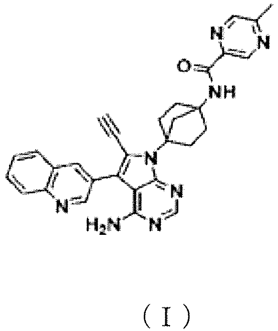

Specifically, the present invention relates to a type II crystal of Compound (1) in free form, a type III crystal of Compound (1) in free form, a type III crystal of Compound (1) with succinic acid, and a type I crystal of Compound (1) with sorbic acid. Herein, the designations type I, type II, type III, etc. are used to conveniently distinguish the crystalline forms, and the crystals of the present invention should not be limited by these designations.

Herein, a crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1)) with an acid refers to a crystal of a salt or a co-crystal with an acid. A crystal of a salt is a crystal in which Compound (1) and a molecule of an acid are bound by ionic bonding, while a co-crystal is a crystal in which Compound (1) and a molecule of an acid are bound by non-ionic interaction. According to the present invention, a crystal of Compound (1) with an acid may be a crystal of a salt or a co-crystal, and both meanings are included. For example, a type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with succinic acid refers to a type III crystal of succinate salt of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide or a type III co-crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with succinic acid. According to an embodiment of the present invention, a type III co-crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with succinic acid and a method for producing the same, or a type I co-crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with sorbic acid and a method for producing the same are provided.

A crystal is a solid whose atoms and molecules are arranged to have a regular repeating structure, which is different from an amorphous (non-crystalline) solid that has no repeating structure. Crystalline or amorphous solids can be examined by powder X-ray diffraction measurement (XRD measurement), differential scanning calorimetry (DSC measurement), simultaneous thermogravimetric-differential thermal analysis (TG-DTA), single crystal analysis, and by other methods. Crystalline polymorphs are those formed from the same types of molecules but with different arrangements of atoms and molecules in the crystals, and the peaks obtained by XRD measurements are known to be different among the crystalline polymorphs. It is also known that solubility, oral absorption, and/or stability are different among the crystalline polymorphs.

Herein, the terms "crystal (crystalline)" and "amorphous" are used in the usual sense, and whether or not it is a crystal can be confirmed from its X-ray diffraction spectrum.

Due to the nature of the data, the diffraction angles and the overall pattern are important in the powder X-ray diffraction patterns in determining the identity of the crystals. The relative intensities of the X-ray powder diffraction patterns should not be strictly interpreted, since they may vary somewhat depending on the direction of crystal growth, particle size, measurement conditions, maintenance of the measurement equipment, and preparation method of the samples to be measured. Herein, a "powder X-ray diffraction spectrum substantially identical to the powder X-ray diffraction spectrum shown in a figure" refers to a powder X-ray diffraction spectrum that can be recognized by a person skilled in the art, taking slight variations in peak positions and intensities into account, as identical to the powder X-ray diffraction spectrum shown in the figure. For example, the value of the diffraction angle (2θ) may have a measurement error in the range of ±0.2°.

When the term "Compound (1)" is simply stated herein, it refers to N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide, and can be used to mean both "crystalline" and "amorphous" solids.

When the term "crystal of Compound (1)" is used herein, it means any of the crystals of Compound (1) in free form and the crystals of Compound (1) with an acid (crystals of a salt of Compound (1) and co-crystals of Compound (1)).

Herein, a crystal for which molecules constituting the crystal other than Compound (1) (other molecules constituting the salt or the co-crystal) are not specified refers to a crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide in free form, which is not limited to a single type of crystalline form (e.g., type I crystal in free form), but may comprise multiple types of crystals in free form.

As used herein, the term "equivalent" means molar equivalent.

In a crystal of Compound (1) with an acid (a crystal of a salt or a co-crystal of Compound (1)), the equivalent of the acid to Compound (1) can be analyzed, for example, by NMR or ion chromatography. Herein, a crystal may be either a hydrate or an anhydrate.

The present invention also comprises a labeled form of Compound (1) or a salt of Compound (1), specifically, a compound in which one or more atoms of Compound (1) or a salt of Compound (1) are replaced by radioactive or non-radioactive isotopes.

The crystal may be any crystal as long as it contains a crystal of Compound (1) and may be a single crystal or otherwise a polymorphic mixture containing a crystal of Compound (1). Specifically, the crystal may have a purity of 40% by weight or more (i.e., 40% by weight or more of it is a single crystal). The crystal preferably has a purity of 50% by weight or more (i.e., 50% by weight or more of it is a single crystal), the crystal more preferably has a purity of 75% by weight or more (i.e., 75% by weight or more of it is a single crystal), the crystal still more preferably has a purity of 90% by weight or more (i.e., 90% by weight or more of it is a single crystal), the crystal yet still more preferably has a purity of 95% by weight or more (i.e., 95% by weight or more of it is a single crystal), the crystal still more preferably has a purity of 98% by weight or more (i.e., 98% by weight or more of it is a single crystal), and the crystal particularly preferably has a purity of 99% by weight or more (i.e., 99% by weight or more of it is a single crystal). The above purity can be determined by differential scanning calorimetry (DSC) or other analyses.

Chemical purity as used herein refers to the purity determined by high-performance liquid chromatography (HPLC) and therefore when the chemical purity of Compound (1) is mentioned, it refers to the purity of Compound (1) as determined by HPLC. In this case, the wavelength of the detector used for the purity determination can be set appropriately. Specifically, the chemical purity of the crystal of Compound (1) is preferably 90% or higher, more preferably 95% or higher, still more preferably 98% or higher, and yet still more preferably 99% or higher.

The values obtained from various patterns may have some errors depending on the direction of crystal growth, particle size, measurement conditions, and so on. Therefore, the values of the diffraction angles (2θ) in the powder X-ray diffraction patterns herein may have measurement errors in the range of ±0.2°. Specifically, "diffraction angle (2θ ± 0.2°)" as used herein means that the value of the diffraction angle (2θ) allows a measurement error of ±0.2°. For example, when the diffraction angle (2θ ± 0.2°) is "6.6°," it means that a diffraction angle of 6.6° ± 0.2° is allowed, which includes the diffraction angles of "6.4° to 6.8°." This value can be determined by the Bragg formula (2d sinθ = nλ), which varies depending on the measured wavelength. In other words, it is possible to convert the value to a diffraction angle at another measured wavelength by substituting the measured wavelength into the above formula. For example, if the values of the diffraction angles (20) are 6.6° and 8.8° at a wavelength λ of 1.54Å, i.e., characteristic X-ray wavelength of CuKα, and the values of d and n are constant, the diffraction angles (2θ) at 0.75Å will be 3.2° and 4.3°, respectively.

Compound (1) used in the crystallization method of the present invention may be, for example, one produced by a method described herein. For crystallization, it is possible to use the synthesized Compound (1) without extracting it as a crystal or after extracting it once as a crystal (crude crystal).

Herein, "room temperature" usually indicates from about 18°C to about 25°C.

Herein, a "lower alcohol" refers to an alcohol with 1 to 5 carbon atoms, which may have either a linear or branched chain. Examples of such a lower alcohol include methanol, ethanol, propanol, isopropyl alcohol, butanol, and pentanol.

The endothermic peak in the simultaneous thermogravimetric-differential thermal analysis (TG-DTA) curve usually allows ±5.0°C since the measured temperature may vary depending on the range of temperature rise per minute, chemical purity of the sample, etc. Therefore, when the peak (onset value) of a crystal of the present invention is measured by TG-DTA, an error of ±5.0°C is considered. The term "around" used in this case means ±5.0°C.

An embodiment of the present invention relates to crystals of Compound (1) in free forms (type II and type III crystals), a crystal of Compound (1) with succinic acid (type III crystal), and a crystal of Compound (1) with sorbic acid (type I crystal). The present inventors have found that among the crystals of Compound (1) in free forms, type II and type III crystals in free forms exhibit stability and can be obtained in a highly reproducible manner, and that the type II crystal in free form further has advantageous properties for manufacturing pharmaceutical products, such as low hygroscopicity and good oral absorption. Furthermore, among crystalline forms or co-crystals with a given acid, crystals with succinic acid and crystals with sorbic acid were found to exhibit stability, low hygroscopicity, high reproducibility, and good oral absorption.

Herein, the stability of the crystals can be evaluated, for example, by the solid stability test described in Test Example 1 below. The change in the chemical purity of a crystal of Compound (1) according to an embodiment of the invention after 4 weeks of storage under the conditions described in Test Example 1 is preferably ±0.15% or less, more preferably ±0.10% or less, and still more preferably ±0.05% or less.

Herein, the hygroscopicity of the crystals can be evaluated by the dynamic vapor sorption (DVS) test described in Test Example 2 below. A crystal of Compound (1) according to an embodiment of the invention preferably has a weight gain of less than 5%, more preferably less than 3%, and still more preferably less than 1% when evaluated under the conditions described in Test Example 2.

Herein, the oral absorption of the crystals can be evaluated by the plasma concentration determination test described in Test Example 5 below.

An embodiment of the present invention provides an antitumor agent comprising the above crystal of Compound (1) (i.e., crystal of Compound (1) in free form or crystal of Compound (1) with an acid (crystal of salt or co-crystal)). An embodiment of the present invention also provides a method for treating a tumor, the method comprising administering an effective amount of the above crystal of Compound (1) (i.e., crystal of Compound (1) in free form or crystal of Compound (1) with an acid (crystal of salt or co-crystal)) to a subject in need thereof. An embodiment of the present invention provides use of the above crystal of Compound (1) (i.e., crystal of Compound (1) in free form or crystal of Compound (1) with an acid (crystal of salt or co-crystal)) for the manufacturing of an antitumor agent. Furthermore, an embodiment of the present invention provides the above crystal of Compound (1) (i.e., crystal of Compound (1) in free form or crystal of Compound (1) with an acid (crystal of salt or co-crystal)) for use in the treatment of a tumor.

A type III crystal of Compound (1) in free form according to an embodiment of the present invention has advantageous properties for manufacturing pharmaceutical products, such as excellent reproducibility and solid stability, compared to other salts or other crystals of Compound (1). In addition, the crystal is also useful as an intermediate for a crystal of the present invention (other type of crystal in free form, crystal with an acid) as shown in the test studies of the crystals obtained in the examples, reference example, and comparative examples.

A type II crystal of Compound (1) in free form, a type III crystal of Compound (1) with succinic acid, and a type I crystal of Compound (1) with sorbic acid according to an embodiment of the present invention have advantageous properties for manufacturing pharmaceutical products, such as lower hygroscopicity, and excellent reproducibility, solid stability, or oral absorption compared to other salts or other crystals of Compound (1).

### N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1))

Compound (1) is a compound having the following structure, which is disclosed in Patent Literature 1 as a compound having an excellent inhibitory effect against EGFR.

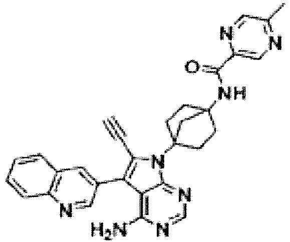

Compound (1) used in the present invention is not particularly limited. For example, one produced by the following production method can be used.

### <Method for producing Compound (1)>

Compound (1) can be produced, for example, by the method described in Patent Literature 1 or the method described herein. Alternatively, the compound can also be produced by combining known reactions using the intermediate described in Patent Literature 1 and herein as a starting material. The product obtained in each step can be subjected to the subsequent step with or without being isolated and purified by a known isolation/purification method, such as concentration, concentration under reduced pressure, crystallization, solvent extraction, reprecipitation, chromatography, or the like. Moreover, in the following production methods, introduction of a protective group or deprotection may be conducted as needed, whether or not it is described, and the order of the steps may be changed as appropriate.

Here, a method for producing Compound (1) using tert-butyl(4-(4-amino-6-bromo-5-(quinolin-3-yl)-7H-pyrrolo[2,3-d]pyrimidin-7-yl)bicyclo[2.2.1]heptan-1-yl)carbamate as a starting material will be described.

### (Step 1)

### Synthesis of tert-butyl(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)carbamate

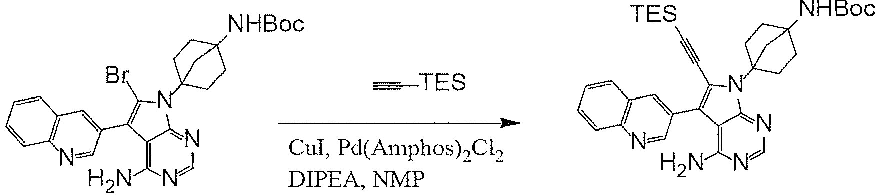

This compound can be obtained by Sonogashira coupling of a TES (triethylsilyl)-protected acetylene derivative with tert-butyl(4-(4-amino-6-bromo-5-(quinolin-3-yl)-7H-pyrrolo[2,3-d]pyrimidin-7-yl)bicyclo[2.2.1]heptan-1-yl)carbamate obtained by the method described in Patent Literature 1 or by other known method.

A catalyst can be used for this reaction. For example, a palladium catalyst can be used alone or in combination with an activator if necessary.

Examples of the palladium catalyst include, but are not particularly limited to, carbon-supported palladium(Pd-C), alumina-supported palladium, silica gel-immobilized palladium, palladium acetate, dichlorobis[di-tert-butyl(4-dimethylaminophenyl)phosphine]palladium(II), tetrakis(triphenylphosphine)palladium(0), dichloro(1,1-bis(diphenylphosphino)ferrocene)palladium(II), and dichlorobis(triphenylphosphine)palladium(II). Examples of a ligand of the catalyst include, but are not particularly limited to, tri(o-tolyl)phosphine, tri(tert-butyl)phosphine, triphenylphosphine, dichlorobis(triphenylphosphine), 2-di-tert-butylphosphino-2',4',6'-triisopropylbiphenyl, 2-dicyclohexylphosphino-2',6'-dimethoxybiphenyl, [4-(N,N-dimethylamino)phenyl]di-tert-butylphosphine, di-tert-butyl(4-dimethylaminophenyl)phosphine, tricyclohexylphosphine, 4,5-bis(diphenylphosphino)-9,9-dimethylxanthene (Xantphos), and bis(2-(diphenylphosphino)phenyl)ether. The ligand of the catalyst can be either coordinated with the palladium catalyst in advance, or the palladium catalyst and the ligand of the catalyst can be added separately to a solvent to be coordinated in the reaction solvent.

While examples of the palladium catalyst are listed above, it is preferably dichlorobis[di-tert-butyl(4-dimethylaminophenyl)phosphine]palladium(II). In addition, the ligand of said catalyst is preferably di-tert-butyl(4-dimethylaminophenyl)phosphine or tricyclohexylphosphine.

As the activator, a copper catalyst, a silver catalyst, a quaternary ammonium, an aqueous amine solution, or the like can be used. Examples of the copper catalyst include salts with monovalent copper ions, specifically copper(I) halides (copper(I) fluoride, copper(I) chloride, copper(I) bromide, copper(I) iodide, copper(I) trifluoromethanesulfonate, etc.). Examples of the silver catalyst include salts with monovalent silver ions, specifically silver(I) oxide. Examples of the quaternary ammonium include quaternary ammonium hydroxides (tetra-n-butylammonium hydroxide: TBAOH, tetraethylammonium hydroxide: TEAOH, n-hexadecyltrimethylammonium hydroxide, choline hydroxide, etc.). As the aqueous amine solution, an aqueous solution of ammonia, a primary amine, a secondary amine, a tertiary amine, or the like can be used. The activator is preferably a copper catalyst, more preferably a salt with a monovalent copper ion, still more preferably copper(I) bromide, copper(I) iodide, or copper(I) trifluoromethanesulfonate, and particularly preferably copper(I) iodide.

The base used for Sonogashira coupling is not particularly limited as long as the base can result the introduction of alkyne groups, and examples thereof include organic bases such as trimethylamine, diethylamine, diisopropylamine (DIPEA), triethylamine (TEA), N-methylmorpholine, diazabicyclononene (DBN), pyridine, 4-dimethylaminopyridine (DMAP), pyridine, morpholine, quinoline, piperidine, and diazabicycloundecene (DBU), and inorganic bases such as alkali metal hydroxides (lithium hydroxide, sodium hydroxide, potassium hydroxide, etc.), alkaline earth metal hydroxides (barium hydroxide, calcium hydroxide, calcium acetate, etc.), alkali metal carbonates (sodium bicarbonate, sodium carbonate, potassium carbonate, cesium carbonate, etc.), alkaline earth metal carbonates (barium carbonate, calcium carbonate), alkali metal phosphates (sodium phosphate, potassium phosphate, etc.), and acetates (magnesium acetate, potassium acetate, calcium acetate, cesium acetate, etc.). Preferably, the base is an organic base and more preferably diisopropylethylamine.

The solvent used for Sonogashira coupling is not particularly limited as long as the solvent is capable of carrying out the above reaction, and examples thereof include N,N-diisopropylethylamine, N-methyl-2-pyrrolidone, dimethyl sulfoxide, N,N-dimethylformamide, N,N-dimethylacetamide, tetrahydrofuran, ethyl acetate, dichloromethane, chloroform, tetrahydrofuran 1,4-dioxane, and toluene. Preferably, the solvent is N-methyl-2-pyrrolidone, N,N-dimethylformamide, or N,N-dimethylacetamide.

The reaction time for Sonogashira coupling is not particularly limited as long as it can result the introduction of alkyne groups. The reaction time can be set as desired. For example, the reaction time is 0.5 to 8 hours, preferably 0.5 to 5 hours, and more preferably 1 to 3 hours.

The reaction temperature for Sonogashira coupling is not particularly limited as long as it can result the introduction of alkyne groups. The reaction temperature can be set as desired. For example, the reaction temperature is 25°C to 120°C, preferably 50°C to 110°C, and more preferably 80°C to 100°C.

### (Step 2)

### Synthesis of 7-(4-aminobicyclo[2.2.1]heptan-1-yl)-5-(quinolin-3-yl)-6-((triethylsilyl)ethynyl)-7H-pyrrolo[2,3-d]pyrimidin-4-amine

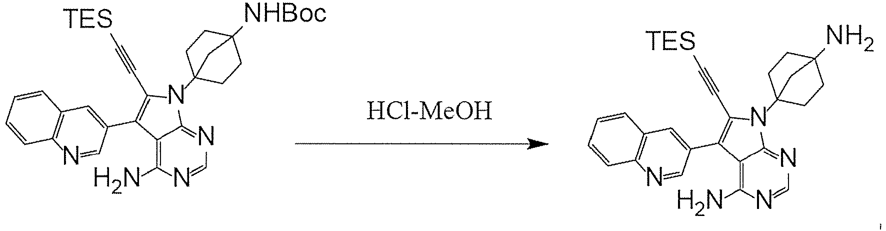

This compound can be obtained by the deprotection of the protective group, Boc (tert-butoxycarbonyl), in tert-butyl(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)carbamate synthesized as described above.

Deprotection of the protective group of amine can be performed by a known method according to the protective group. The deprotection can be performed under acidic conditions, under reductive conditions, under basic conditions, using a palladium catalyst, or the like. For example, in the case of Boc, deprotection is generally carried out under acidic conditions, and any acid can be used to provide the acidic conditions as long as the deprotection can proceed. Preferably, the acid is trifluoroacetic acid, hydrochloric acid, or sulfuric acid. Moreover, the amount of the acid used to provide the acidic condition is not particularly limited as long as the reaction can proceed.

The solvent used for the deprotection reaction of the protective group of amine is not particularly limited as long as the reaction can proceed. The solvent can be selected from the same solvents as those used in step of deprotecting a silicon-linked protective group described below. Preferably, the solvent is methanol, ethanol, 2-propanol, acetonitrile, or tetrahydrofuran, more preferably methanol, ethanol, or 2-propanol, and still more preferably methanol. While the amount of the solvent used is not particularly limited as long as the reaction can proceed, it is preferably 1 to 100 L, more preferably 5 to 50 L, and still more preferably 10 to 30 L per 1 kg of the reactant.

### (Step 3)

### Synthesis of N-(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)-5-methylpyrazine-2-carboxamide

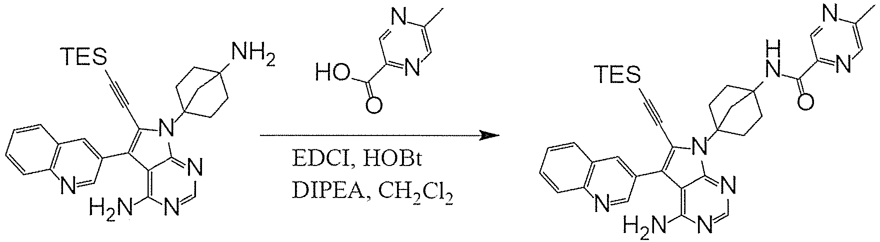

This compound can be obtained by condensing 7-(4-aminobicyclo[2.2.1]heptan-1-yl)-5-(quinolin-3-yl)-6-((triethylsilyl)ethynyl)-7H-pyrrolo[2,3-d]pyrimidin-4-amine obtained in Step 2 with 5-methylpyrazine-2-carboxylic acid.

The condensing agent used for the condensation reaction is not particularly limited as long as the reaction can proceed. Examples of the condensing agent include: combinations of a carbodiimide condensing agent such as 1-(3-dimethylaminopropyl)-3-ethylcarbodiimide or a hydrochloride thereof (WSC or EDC), dicyclohexylcarbodiimide (DCC), 1-cyclohexyl-3-(2-morpholinoethyl)carbodiimide metho-p-toluenesulfonate (CMC), diisopropylcarbodiimide (DIC), or 1,3-bis(2,2-dimethyl-1,3-dioxolan-4-ylmethyl)carbodiimide (BDDC) and an activated ester-forming alcohol such as 1-hydroxybenzotriazole (HOBt), 1-hydroxy-7-azabenzotriazole (HOAt), ethyl(hydroxyimino)cyanoacetate (Oxyma), N-hydroxysuccinimide (HOSu), or pentafluorophenol; uronium condensing agents such as O-(7-azabenzotriazolo-1-yl)-N,N,N',N'-tetramethylhexauronium hexafluorophosphate (HATU) and (1-cyano-2-ethoxy-2-oxoethylideneaminooxy)dimethylaminomorpholinocarbenium hexafluorophosphate (COMU); 2-halo-N-alkylpyridinium salts (Mukaiyama condensation reagent) such as 2-chloro-6-methyl-1,3-diphenylpyridinium tetrafluoroborate and 1-methyl-2-chloropyridinium iodide; imidazole condensing agents such as N,N'-carbonyldiimidazole (CDI) and 1,1'-carbonyldi(1,2,4-triazole) (CDT); phosphonium condensing agents such as bromotripyrrolidinophosphonium hexafluorophate, chlorotripyrrolidinophosphonium hexafluorophate, 1H-benzotriazol-1-yloxytris(dimethylamino)phosphonium hexafluorophosphate (BOP), (benzotriazol-1-yloxy)tripyrrolidinophosphonium hexafluorophosphate (PyBOP), and (7-azabenzotriazol-1-yloxy)tripyrrolidinophosphonium hexafluorophosphate (PyAOP); triazine condensing agents such as 4-(4,6-dimethoxy-1,3,5-triazin-2-yl)-4-methylmorpholinium chloride (DMTMM) and (4,6-dimethoxy-1,3,5-triazin-2-yl)-(octoxy-2-oxoethyl)dimethylammonium trifluoromethanesulfonate; 2,4,6-tripropyl-1,3,5,2,4,6-trioxatriphosphorinane-2,4,6-trioxide (T3P); and diphenylphosphoryl azide (DPPA). Preferably, the condensing agent is a combination of a carbodiimide condensing agent and an activated ester-forming alcohol, and more preferably a combination of EDC and HOBt. These condensing agents include polymer-supported reagents.

The solvent used for the condensation reaction is not particularly limited as long as the reaction can proceed. The solvent can be selected from the same solvents as those used in the step of deprotecting a silicon-linked protective group. Preferably, the solvent is dichloromethane, chloroform, ethyl acetate, dimethyl sulfoxide, diethyl ether, tetrahydrofuran, 1,4-dioxane, acetone, N,N-dimethylformamide, N-methylpyrrolidone, toluene, or acetonitrile tetrahydrofuran, more preferably, dichloromethane, chloroform, ethyl acetate, dimethyl sulfoxide, or N,N-dimethylformamide, and still more preferably dichloromethane or chloroform. While the amount of the solvent used is not particularly limited as long as the reaction can proceed, it is preferably 5 to 100 L, more preferably 15 to 50 L, and still more preferably 25 to 35 L per 1 kg of the reactant.

### (Step 4)

### Synthesis of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1))

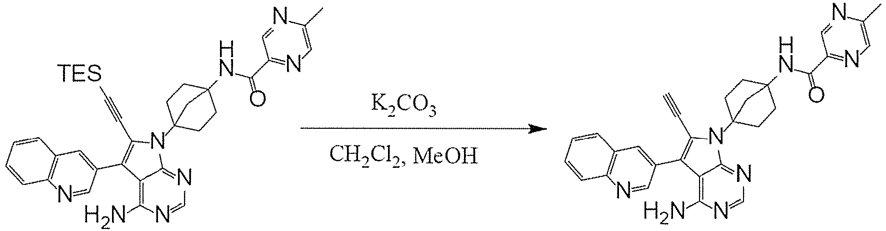

This compound can be obtained by the deprotection of the protective group, TES, of acetylene in N-(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)-5-methylpyrazine-2-carboxamide obtained in Step 3, under basic conditions.

While the reagent used for providing basic conditions is not particularly limited as long as the reaction can proceed, examples thereof include inorganic bases such as metal hydroxides (sodium hydroxide, calcium hydroxide, etc.), metal hydrides (lithium hydride, sodium hydride, etc.), and metal carbonates (sodium carbonate, potassium carbonate, cesium carbonate, calcium carbonate, lithium carbonate, magnesium carbonate, sodium bicarbonate, etc.), and organic bases such as metal alkoxides (sodium methoxide, potassium tert-butoxide, etc.), metal amides (sodium amide, lithium diisopropyl amide, etc.), alkyl metal compounds (n-butyllithium, trimethylaluminum, etc.), alkylamines (triethylamine, tetramethylethylenediamine, piperidine, 1,4-diazabicyclo[2.2.2]octane, etc.), heterocyclic amines (diazabicycloundecene, pyridine, imidazole, etc.), and quaternary ammonium fluorides (tetra-n-butylammonium fluoride). Preferably, the reagent used for providing basic conditions is a reagent that does not contain fluoride ions, more preferably a metal carbonate, and still more preferably potassium carbonate. These reagents can be used alone or in combination to adjust the pH to the desired level.

While the amount of the reagent used is not particularly limited as long as the reaction can proceed, it can be used in an amount of, for example, 0.1 to 50 moles per mole of the starting compound (compound represented by formula (II)). Preferably, it is 0.1 to 10 moles, and more preferably 0.1 to 2 moles.

The solvent used for the deprotection step is not particularly limited as long as the pH can be set to a basic condition and the compound used in the reaction can be dissolved. Examples of the solvent include a C5-C10 hydrocarbon, a C6-C14 aromatic hydrocarbon, a C1-C6 alcohol, a C3-C10 aliphatic carboxylic ester, a C3-C10 ketone, a C4-C10 ether, a non-protic polar organic solvent, and a mixed solvent thereof. Herein, C followed by a number refers to the number of carbon atoms, which is the total number of carbon atoms contained in the compound.

C5-C10 hydrocarbons refer to hydrocarbons with 5 to 10 carbon atoms, such as pentane, hexane, heptane, octane, nonane, decane, cyclopentane, cyclohexane, cycloheptane, cyclooctane, cyclononane, and cyclodecane.

C6-C14 aromatic hydrocarbons refer to aromatic hydrocarbons with 6 to 14 carbon atoms, such as benzene, naphthalene, anthracene, toluene, xylene, cumene, styrene, and phenanthrene.

C1-C6 alcohols refer to alcohols with 1 to 6 carbon atoms, such as methanol, ethanol, n-propanol, isopropanol, n-butanol, tert-butanol, n-pentanol, and n-hexanol.

C3-C10 aliphatic carboxylic esters refer to aliphatic carboxylic esters with 3 to 10 carbon atoms, such as propyl formate, methyl acetate, ethyl acetate, propyl acetate, methyl propionate, ethyl propionate, propyl propionate, methyl butanoate, and ethyl butanoate.

C3-C10 ketones refer to ketones with 3 to 10 carbon atoms, such as acetone, ethyl methyl ketone, diethyl ketone, isopropyl methyl ketone, and cyclohexanone.

C4-C10 ethers refer to ethers with 4 to 10 carbon atoms, such as diethyl ether, tert-butyl methyl ether, tetrahydrofuran, and 1,4-dioxane.

Non-protic polar organic solvents refer to solvents that do not have a proton to ionize, such as N,N-dimethylformamide, N,N-dimethylacetamide, N-methylpyrrolidone, chloroform, and dichloromethane.

When Compound (1) is produced according to Steps 1-4 described here, a type III crystal of Compound (1) can be formed.

### Type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1)) in free form

According to an embodiment of the present invention, there is provided a type III crystal of Compound (1) in free form. A type III crystal of Compound (1) in free form according to an embodiment of the present invention has a powder X-ray diffraction spectrum shown in Figure 1 or a powder X-ray diffraction spectrum substantially identical thereto.

Here, examples of characteristic peaks in the powder X-ray diffraction spectrum of the type III crystal of Compound (1) in free form according to an embodiment of the present invention include those at one or more diffraction angles (2θ ± 0.2°) selected from 6.6°, 8.8°, 11.6°, 12.3°, 13.8°, 17.9°, 18.6°, 21.3°, and 22.3°.

A type III crystal of Compound (1) in free form according to an embodiment of the present invention has peaks in a powder X-ray diffraction spectrum at diffraction angles (2θ ± 0.2°) of:
(i) one or more selected from the group consisting of 8.8°, 11.6°, and 17.9°; and
(ii) two or more selected from the group consisting of 6.6°, 12.3°, 13.8°, 18.6°, 21.3°, and 22.3°. Specifically, the crystal has peaks at a total of three or more selected from groups (i) and (ii) above. A type III crystal of Compound (1) in free form according to a preferred embodiment of the present invention has peaks in a powder X-ray diffraction spectrum at a total of four or more, preferably a total of five or more, still more preferably a total of six or more, and yet still more preferably seven or more diffraction angles (2θ ± 0.2°) selected from groups (i) and (ii) above. According to another embodiment of the present invention, a type III crystal of Compound (1) in free form may have peaks in a powder X-ray diffraction spectrum at a total of eight or all diffraction angles (2θ ± 0.2°) selected from groups (i) and (ii) above. According to another embodiment, a type III crystal of Compound (1) in free form may have peaks at two or more, three or more, or all selected from group (i) above. According to yet another embodiment, a type III crystal of Compound (1) in free form may have peaks at three or more, four or more, five or more, or all selected from group (ii) above. According to yet another embodiment, the crystal may have additional peaks other than those above at one or more diffraction angles (2θ ± 0.2°) mentioned in the examples.

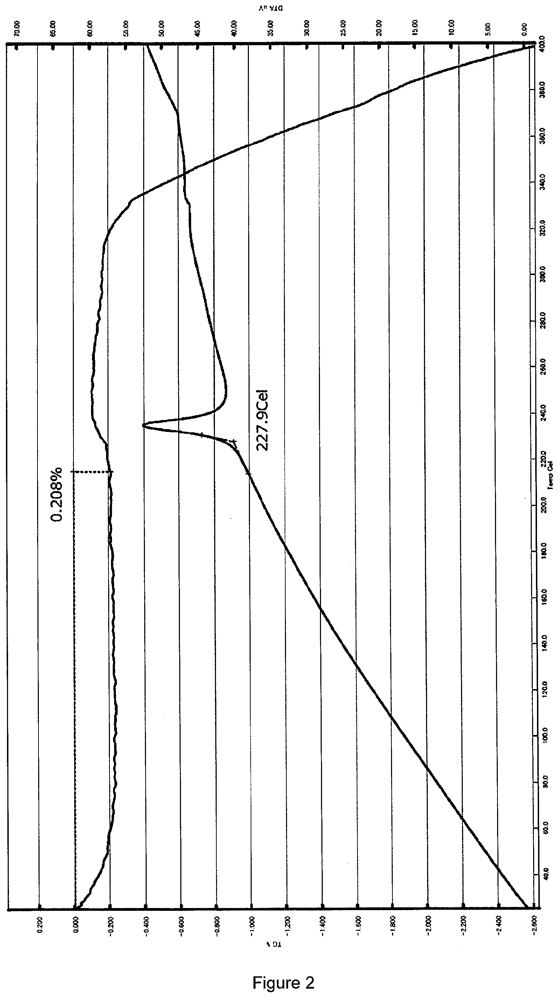

A type III crystal of Compound (1) in free form according to an embodiment of the present invention has an exothermic peak (onset value) determined by simultaneous thermogravimetric-differential thermal analysis at 223°C to 233°C, in other words, around 228°C. According to another embodiment, a type III crystal of Compound (1) in free form has the simultaneous thermogravimetric-differential thermal analysis (TG-DTA) curve shown in Figure 2.

A type III crystal of Compound (1) in free form according to an embodiment of the present invention has peaks in a powder X-ray diffraction spectrum at diffraction angles (2θ ± 0.2°) of:
(i) one or more selected from the group consisting of 8.8°, 11.6°, and 17.9°; and
(ii) two or more selected from the group consisting of 6.6°, 12.3°, 13.8°, 18.6°, 21.3°, and 22.3°,
and an exothermic peak (onset value) determined by simultaneous thermogravimetric-differential thermal analysis at around 228°C. A type III crystal of Compound (1) in free form according to a preferred embodiment of the present invention is a crystal that has peaks in a powder X-ray diffraction spectrum at a total of four or more diffraction angles (2θ ± 0.2°) selected from groups (i) and (ii) above and that has an exothermic peak (onset value) determined by simultaneous thermogravimetric-differential thermal analysis at around 228°C, preferably a crystal that has peaks at five or more diffraction angles (2θ ± 0.2°) selected from groups (i) and (ii) above and that has an exothermic peak (onset value) determined by simultaneous thermogravimetric-differential thermal analysis at around 228°C, more preferably a crystal that has peaks at six or more diffraction angles (2θ ± 0.2°) selected from groups (i) and (ii) above and that has an exothermic peak (onset value) determined by simultaneous thermogravimetric-differential thermal analysis at around 228°C, and still more preferably a crystal that has peaks at seven or more diffraction angles (2θ ± 0.2°) selected from groups (i) and (ii) above and that has an exothermic peak (onset value) determined by simultaneous thermogravimetric-differential thermal analysis at around 228°C. According to another embodiment of the present invention, a type III crystal of Compound (1) in free form may have peaks in a powder X-ray diffraction spectrum at a total of eight or all diffraction angles (2θ ± 0.2°) selected from groups (i) and (ii) above, and may have an exothermic peak (onset value) determined by simultaneous thermogravimetric-differential thermal analysis at around 228°C. According to another embodiment, a type III crystal of Compound (1) in free form may have peaks at two or more selected from group (i) above and may have an exothermic peak (onset value) determined by simultaneous thermogravimetric-differential thermal analysis at around 228°C, may have peaks at three or more selected from group (i) above and may have an exothermic peak (onset value) determined by simultaneous thermogravimetric-differential thermal analysis at around 228°C, or may have peaks at all selected from group (i) above and may have an exothermic peak (onset value) determined by simultaneous thermogravimetric-differential thermal analysis at around 228°C. A type III crystal of Compound (1) in free form according to yet another embodiment of the present invention may have peaks at three or more selected from group (ii) above and may have an exothermic peak (onset value) determined by simultaneous thermogravimetric-differential thermal analysis at around 228°C, may have peaks at four or more selected from group (ii) above and may have an exothermic peak (onset value) determined by simultaneous thermogravimetric-differential thermal analysis at around 228°C, may have peaks at five or more selected from group (ii) above and may have an exothermic peak (onset value) determined by simultaneous thermogravimetric-differential thermal analysis at around 228°C, or may have peaks at all selected from group (ii) above and may have an exothermic peak (onset value) determined by simultaneous thermogravimetric-differential thermal analysis at around 228°C.

### (Method for producing type III crystal of Compound (1) in free form)

A type III crystal of Compound (1) in free form according to an embodiment of the present invention can be obtained, for example, by stirring Compound (1) in a methanol-containing solvent, such as a mixed solvent of water and methanol. Apart from a mixed solvent of methanol and water, examples of the methanol-containing solvent include a combination of methanol and N-methyl-2-pyrrolidone. Preferably, it is a mixed solvent of methanol and N-methyl-2-pyrrolidone.

While the amount of the solvent is not particularly limited, it is preferably 5 to 50 (volume/weight) times, more preferably 5 to 30 (volume/weight) times, and still more preferably 5 to 10 (volume/weight) times the amount of Compound (1).

The stirring time can be set as desired in a range from 10 minutes to 120 hours. The stirring time is preferably 1 to 24 hours, more preferably 1 to 5 hours, and still more preferably 1 to 3 hours.

While the stirring temperature can be set as desired, it should be less than about 60°C. It is preferably 10°C to 50°C, more preferably 20°C to 40°C, and still more preferably 20°C to 30°C. In order to remove the solvent used, drying may be performed by any known drying method, such as drying under reduced pressure. According to this method, a type III crystal of Compound (1) in free form can be readily mass-produced.

### Type II crystal of N-(4-(4-amino-6-ethynyl-5-(quinolin-3-yl)-7H-pyrrolo[2,3-dipyrimidin-7-yl)bicyclo[2.2.1]heptan-1-yl)-5-methylpyrazine-2-carboxamide (Compound (1)) in free form

According to an embodiment of the present invention, there is provided a type II crystal of Compound (1) in free form. A type II crystal of Compound (1) in free form according to an embodiment of the present invention has a powder X-ray diffraction spectrum shown in Figure 3 or a powder X-ray diffraction spectrum substantially identical thereto.

Here, examples of characteristic peaks in the powder X-ray diffraction spectrum of the type II crystal of Compound (1) in free form according to an embodiment of the present invention include those at one or more diffraction angles (20 ± 0.2°) selected from 7.8°, 10.6°, 14.0°, 14.8°, 15.6°, 17.2°, 20.1°, 21.6°, 22.2°, 24.7°, 25.8°, and 28.9°.

A type II crystal of Compound (1) in free form according to an embodiment of the present invention has peaks in a powder X-ray diffraction spectrum at diffraction angles (2θ ± 0.2°) of:
(i) one or more selected from the group consisting of 15.6°, 20.1°, 24.7°, and 28.9°; and
(ii) two or more selected from the group consisting of 7.8°, 10.6°, 14.0°, 14.8°, 17.2°, 21.6°, 22.2°, and 25.8°. Specifically, the crystal has peaks at a total of three or more selected from groups (i) and (ii) above. A type II crystal of Compound (1) in free form according to a preferred embodiment of the present invention has peaks in a powder X-ray diffraction spectrum at a total of four or more, preferably a total of five or more, still more preferably a total of six or more, and yet still more preferably seven or more diffraction angles (2θ ± 0.2°) selected from groups (i) and (ii) above. According to another embodiment of the present invention, a type II crystal of Compound (1) in free form may have peaks in a powder X-ray diffraction spectrum at a total of eight, nine, ten, eleven, or all diffraction angles (2θ ± 0.2°) selected from groups (i) and (ii) above. According to another embodiment, a type II crystal of Compound (1) in free form may have peaks at two or more, three or more, or all selected from group (i) above. According to yet another embodiment, a type II crystal of Compound (1) in free form may have peaks at three or more, four or more, five or more, six or more, seven or more, or all selected from group (ii) above. According to yet another embodiment, the crystal may have additional peaks other than those above at one or more diffraction angles (2θ ± 0.2°) mentioned in the examples.

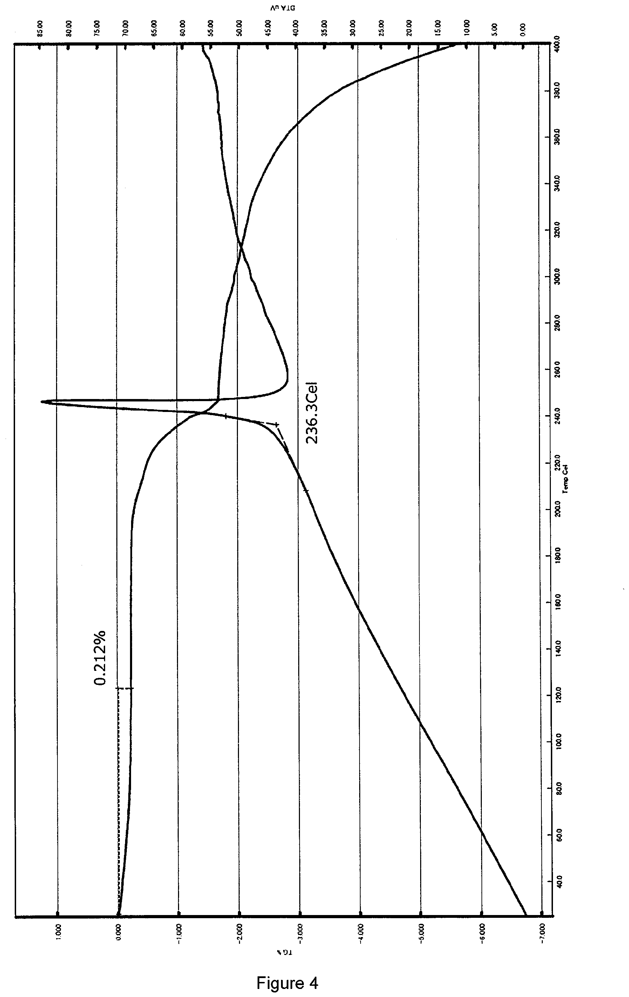

A type II crystal of Compound (1) in free form according to an embodiment of the present invention has an exothermic peak (onset value) determined by simultaneous thermogravimetric-differential thermal analysis at 231°C to 241°C, in other words, around 236°C. According to another embodiment, a type II crystal of Compound (1) in free form has the simultaneous thermogravimetric-differential thermal analysis (TG-DTA) curve shown in Figure 4.

A type II crystal of Compound (1) in free form according to an embodiment of the present invention has peaks in a powder X-ray diffraction spectrum at diffraction angles (2θ ± 0.2°) of:
(i) one or more selected from the group consisting of 15.6°, 20.1°, 24.7°, and 28.9°; and
(ii) two or more selected from the group consisting of 7.8°, 10.6°, 14.0°, 14.8°, 17.2°, 21.6°, 22.2°, and 25.8°,
and an exothermic peak (onset value) determined by simultaneous thermogravimetric-differential thermal analysis at around 236°C. A type II crystal of Compound (1) in free form according to a preferred embodiment of the present invention is a crystal that has peaks in a powder X-ray diffraction spectrum at a total of four or more diffraction angles (2θ ± 0.2°) selected from groups (i) and (ii) above and that has an exothermic peak (onset value) determined by simultaneous thermogravimetric-differential thermal analysis at around 236°C, preferably a crystal that has peaks at five or more diffraction angles (2θ ± 0.2°) selected from groups (i) and (ii) above and that has an exothermic peak (onset value) determined by simultaneous thermogravimetric-differential thermal analysis at around 236°C, more preferably a crystal that has peaks at six or more diffraction angles (2θ ± 0.2°) selected from groups (i) and (ii) above and that has an exothermic peak (onset value) determined by simultaneous thermogravimetric-differential thermal analysis at around 236°C, and still more preferably a crystal that has peaks at seven or more diffraction angles (2θ ± 0.2°) selected from groups (i) and (ii) above and that has an exothermic peak (onset value) determined by simultaneous thermogravimetric-differential thermal analysis at around 236°C. According to another embodiment of the present invention, a type II crystal of Compound (1) in free form may have peaks in a powder X-ray diffraction spectrum at a total of eight, nine, ten, eleven, or all diffraction angles (2θ ± 0.2°) selected from groups (i) and (ii) above and may have an exothermic peak (onset value) determined by simultaneous thermogravimetric-differential thermal analysis at around 236°C. According to another embodiment, a type II crystal of Compound (1) in free form may have peaks at two or more selected from group (i) above and may have an exothermic peak (onset value) determined by simultaneous thermogravimetric-differential thermal analysis at around 236°C, may have peaks at three or more selected from group (i) above and may have an exothermic peak (onset value) determined by simultaneous thermogravimetric-differential thermal analysis at around 236°C, or may have peaks at all selected from group (i) above and may have an exothermic peak (onset value) determined by simultaneous thermogravimetric-differential thermal analysis at around 236°C. According to yet another embodiment, a type II crystal of Compound (1) in free form may have peaks at three or more selected from group (ii) above and may have an exothermic peak (onset value) determined by simultaneous thermogravimetric-differential thermal analysis at around 236°C, may have peaks at four or more selected from group (ii) above and may have an exothermic peak (onset value) determined by simultaneous thermogravimetric-differential thermal analysis at around 236°C, may have peaks at five or more selected from group (ii) above and may have an exothermic peak (onset value) determined by simultaneous thermogravimetric-differential thermal analysis at around 236°C, may have peaks at six or more selected from group (ii) above and may have an exothermic peak (onset value) determined by simultaneous thermogravimetric-differential thermal analysis at around 236°C, may have peaks at seven or more selected from group (ii) above and may have an exothermic peak (onset value) determined by simultaneous thermogravimetric-differential thermal analysis at around 236°C, or may have peaks at all selected from group (ii) above and may have an exothermic peak (onset value) determined by simultaneous thermogravimetric-differential thermal analysis at around 236°C.

### (Method for producing type II crystal of Compound (1) in free form)

A type II crystal of Compound (1) in free form according to an embodiment of the present invention can be obtained, for example, by stirring Compound (1) in a solvent such as tetrahydrofuran.

Apart from tetrahydrofuran, examples of solvents that can be used include lower alcohols with 3 or more carbon atoms, acetone, ethyl acetate, and N-methyl-2-pyrrolidone, which can be used either alone or in mixture. Preferably, it is tetrahydrofluorane.

While the amount of the solvent is not particularly limited, it is preferably 5 to 50 (volume/weight) times, more preferably 5 to 30 (volume/weight) times, and still more preferably 5 to 20 (volume/weight) times the amount of Compound (1).

The stirring time can be set as desired in a range from 10 minutes to 120 hours. The stirring time is preferably 2 to 48 hours and more preferably 5 to 24 hours.

While the stirring temperature can be set as desired, it should be less than about 60°C. It is preferably 20°C to 50°C, more preferably 20°C to 30°C, and still more preferably 25°C. In order to remove the solvent used, drying may be performed by any known drying method, such as drying under reduced pressure.

### Type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1)) with succinic acid

According to an embodiment of the present invention, there is provided a type III crystal of Compound (1) with succinic acid. A type III crystal of Compound (1) with succinic acid according to an embodiment of the present invention has a powder X-ray diffraction spectrum shown in Figure 13 or a powder X-ray diffraction spectrum substantially identical thereto.

Here, examples of characteristic peaks in the powder X-ray diffraction spectrum of the type III crystal of Compound (1) with succinic acid according to an embodiment of the present invention include those at one or more diffraction angles (20 ± 0.2°) selected from the group consisting of 10.4°, 13.3°, 14.6°, 15.7°, 17.2°, 18.3°, 19.7°, 20.8°, 22.3°, and 27.5°.

A type III crystal of Compound (1) with succinic acid according to an embodiment of the present invention is a crystal having three or more peaks selected from the above characteristic peaks, preferably a crystal having four or more peaks selected from the above characteristic peaks, more preferably a crystal having five or more peaks selected from the above characteristic peaks, still more preferably a crystal having six or more peaks selected from the above characteristic peaks, and yet still more preferably a crystal having seven or more peaks selected from the above characteristic peaks. According to another embodiment of the present invention, a type III crystal of Compound (1) with succinic acid may have eight or more, nine or more, or all peaks selected from the above characteristic peaks. According to yet another embodiment, the crystal may have additional peaks other than those above at one or more diffraction angles (2θ ± 0.2°) mentioned in the examples.

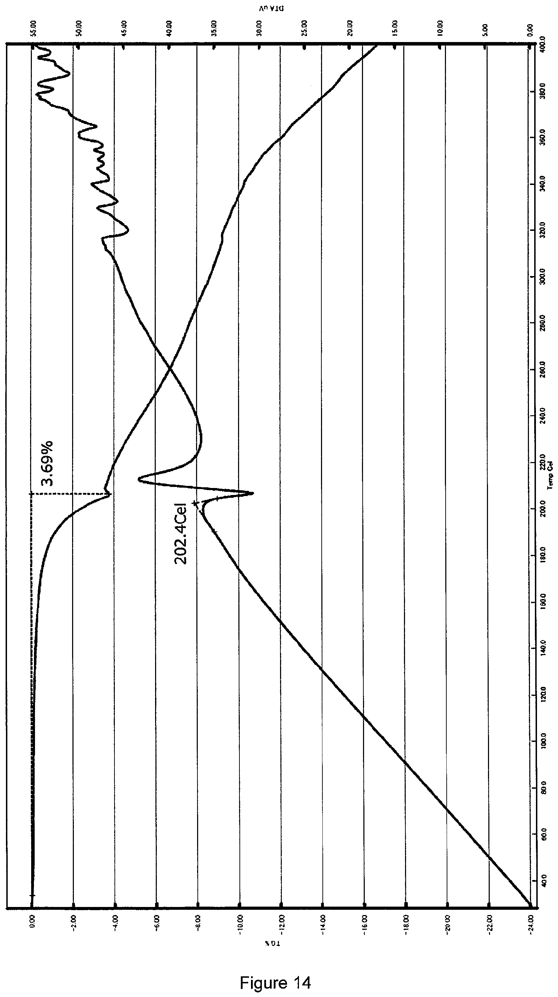

A type III crystal of Compound (1) with succinic acid according to an embodiment of the present invention has an endothermic peak (onset value) determined by simultaneous thermogravimetric-differential thermal analysis at 197°C to 207°C, in other words, around 202°C. According to another embodiment, a type III crystal of Compound (1) with succinic acid has the simultaneous thermogravimetric-differential thermal analysis (TG-DTA) curve shown in Figure 14.

A type III crystal of Compound (1) with succinic acid according to an embodiment of the present invention has characteristic peaks in a powder X-ray diffraction spectrum at one or more diffraction angles (2θ ± 0.2°) selected from the group consisting of 10.4°, 13.3°, 14.6°, 15.7°, 17.2°, 18.3°, 19.7°, 20.8°, 22.3°, and 27.5°, and has an endothermic peak (onset value) determined by simultaneous thermogravimetric-differential thermal analysis at around 202°C. A type III crystal of Compound (1) with succinic acid according to an embodiment of the present invention is a crystal having three or more peaks selected from the above characteristic peaks and having an endothermic peak (onset value) determined by simultaneous thermogravimetric-differential thermal analysis at around 202°C, preferably a crystal having four or more peaks selected from the above characteristic peaks and having an endothermic peak (onset value) determined by simultaneous thermogravimetric-differential thermal analysis at around 202°C, more preferably a crystal having five or more peaks selected from the above characteristic peaks and having an endothermic peak (onset value) determined by simultaneous thermogravimetric-differential thermal analysis at around 202°C, still more preferably a crystal having six or more peaks selected from the above characteristic peaks and having an endothermic peak (onset value) determined by simultaneous thermogravimetric-differential thermal analysis at around 202°C, and yet still more preferably a crystal having seven or more peaks selected from the above characteristic peaks and having an endothermic peak (onset value) determined by simultaneous thermogravimetric-differential thermal analysis at around 202°C. According to another embodiment of the present invention, a type III crystal of Compound (1) with succinic acid may have eight or more, nine or more, or all peaks selected from the above characteristic peaks, and may have an endothermic peak (onset value) determined by simultaneous thermogravimetric-differential thermal analysis at around 202°C.

Herein, a type III crystal of Compound (1) with succinic acid may be a salt or a co-crystal of Compound (1) and succinic acid. Preferably, the equivalent ratio of Compound (1) to succinic acid is 1:1-10, preferably 1:1-5, more preferably 1:1-3, and still more preferably 1:1 or 1:3.

### (Method for producing type III crystal of Compound (1) with succinic acid)

A type III crystal of Compound (1) with succinic acid according to an embodiment of the present invention can be obtained, for example, by stirring Compound (1) and succinic acid in a solvent such as acetonitrile.

Apart from acetonitrile, examples of the solvent used include lower alcohols with 2 or more carbon atoms, methyl ethyl ketone, tetrahydrofuran, and 2-methyl tetrahydrofuran. Preferably, the solvent is acetonitrile, tetrahydrofuran, or 2-methyltetrahydrofuran.

While the amount of the solvent is not particularly limited, it is preferably 5 to 50 (volume/weight) times, more preferably 5 to 20 (volume/weight) times, and still more preferably 5 to 9 (volume/weight) times the amount of Compound (1).

The stirring time can be set as desired, and it is preferably 10 minutes to 24 hours, more preferably 30 minutes to 3 hours, and still more preferably 1 to 2 hours.

While the stirring temperature can be set as desired, it should be less than about 60°C. It is preferably 30°C to 50°C, more preferably 40°C to 50°C, and still more preferably 50°C.

In order to remove the solvent used, drying may be performed by any known drying method, such as drying under reduced pressure.

### Type I crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1)) with sorbic acid

According to an embodiment of the present invention, there is provided a type I crystal of Compound (1) with sorbic acid. A type I crystal of Compound (1) with sorbic acid according to an embodiment of the present invention has a powder X-ray diffraction spectrum shown in Figure 15 or a powder X-ray diffraction spectrum substantially identical thereto.

Here, examples of characteristic peaks in the powder X-ray diffraction spectrum of the type I crystal of Compound (1) with sorbic acid according to an embodiment of the present invention include those at one or more diffraction angles (20 ± 0.2°) selected from the group consisting of 12.6°, 14.7°, 15.4°, 15.9°, 17.1°, 18.9°, 22.2°, 24.1°, 25.8°, and 27.4°.

A type I crystal of Compound (1) with sorbic acid according to an embodiment of the present invention is a crystal having three or more peaks selected from the above characteristic peaks, preferably a crystal having four or more peaks selected from the above characteristic peaks, more preferably a crystal having five or more peaks selected from the above characteristic peaks, still more preferably a crystal having six or more peaks selected from the above characteristic peaks, and yet still more preferably a crystal having seven or more peaks selected from the above characteristic peaks. According to another embodiment of the present invention, a type I crystal of Compound (1) with sorbic acid may have eight or more, nine or more, or all peaks selected from the above characteristic peaks. According to yet another embodiment, the crystal may have additional peaks other than those above at one or more diffraction angles (2θ ± 0.2°) mentioned in the examples.

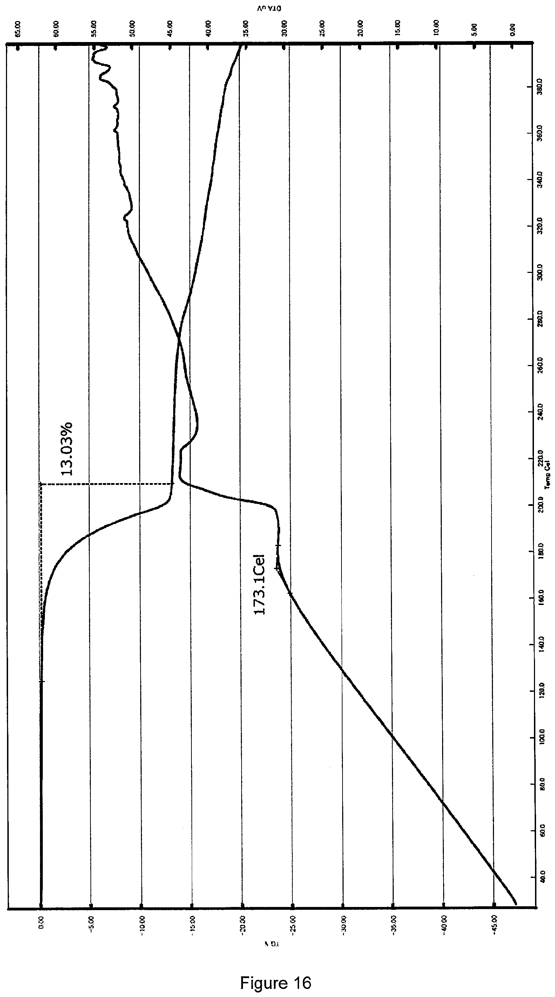

A type I crystal of Compound (1) with sorbic acid according to an embodiment of the present invention has an endothermic peak (onset value) determined by simultaneous thermogravimetric-differential thermal analysis at 168°C to 178°C, in other words, around 173°C. According to another embodiment, a type I crystal of Compound (1) with sorbic acid has the simultaneous thermogravimetric-differential thermal analysis (TG-DTA) curve shown in Figure 16.

A type I crystal of Compound (1) with sorbic acid according to an embodiment of the present invention has characteristic peaks in a powder X-ray diffraction spectrum at one or more diffraction angles (2θ ± 0.2°) selected from the group consisting of 12.6°, 14.7°, 15.4°, 15.9°, 17.1°, 18.9°, 22.2°, 24.1°, 25.8°, and 27.4°, and has an endothermic peak (onset value) determined by simultaneous thermogravimetric-differential thermal analysis at around 173°C. A type I crystal of Compound (1) with sorbic acid according to an embodiment of the present invention is a crystal having three or more peaks selected from the above characteristic peaks and having an endothermic peak (onset value) determined by simultaneous thermogravimetric-differential thermal analysis at around 173°C, preferably a crystal having four or more peaks selected from the above characteristic peaks and having an endothermic peak (onset value) determined by simultaneous thermogravimetric-differential thermal analysis at around 173°C, more preferably a crystal having five or more peaks selected from the above characteristic peaks and having an endothermic peak (onset value) determined by simultaneous thermogravimetric-differential thermal analysis at around 173°C, still more preferably a crystal having six or more peaks selected from the above characteristic peaks and having an endothermic peak (onset value) determined by simultaneous thermogravimetric-differential thermal analysis at around 173°C, and yet still more preferably a crystal having seven or more peaks selected from the above characteristic peaks and having an endothermic peak (onset value) determined by simultaneous thermogravimetric-differential thermal analysis at around 173°C. According to another embodiment of the present invention, a type I crystal of Compound (1) with sorbic acid may have eight or more, nine or more, or all peaks selected from the above characteristic peaks, and may have an endothermic peak (onset value) determined by simultaneous thermogravimetric-differential thermal analysis at around 173°C.

Herein, a type I crystal of Compound (1) with sorbic acid may be a salt or a co-crystal of Compound (1) and sorbic acid. Preferably, the equivalent ratio of Compound (1) to sorbic acid is 1:1-10, preferably 1:1-5, more preferably 1:1-3, and still more preferably 1:1.

### (Method for producing type I crystal of Compound (1) with sorbic acid)

A type I crystal of Compound (1) with sorbic acid according to an embodiment of the present invention can be obtained, for example, by stirring Compound (1) and sorbic acid in acetonitrile.

Apart from acetonitrile, examples of the solvent used include lower alcohols, tetrahydrofuran, acetone, methyl ethyl ketone, water, and ethyl acetate. Preferably, the solvent is acetonitrile, tetrahydrofuran, acetone, methyl ethyl ketone, or water.

While the amount of the solvent is not particularly limited, it is preferably 5 to 50 (volume/weight) times, more preferably 5 to 20 (volume/weight) times, and still more preferably 5 to 10 (volume/weight) times the amount of Compound (1).

The stirring time can be set as desired, and it is preferably 10 minutes to 120 hours, more preferably 1 to 10 hours, and still more preferably 2 to 7.5 hours.

While the stirring temperature can be set as desired, it should be less than about 60°C. It is preferably 30°C to 50°C, more preferably 40°C to 50°C, and still more preferably 50°C.

In order to remove the solvent used, drying may be performed by any known drying method, such as drying under reduced pressure.

### (Purification and other manipulation of crystals)

The precipitated crystal can be isolated and purified from the solution used for dissolving the aforementioned crystal, the solution mixed with the aforementioned crystal, and the like by a known isolation/purification technique such as filtration, washing with water or an organic solvent, or drying under reduced pressure. Examples of the organic solvent used for washing include lower alcohols, acetone, acetonitrile, etc.

Each crystal can also be produced by adding the crystal of interest as a seed crystal to a specific solvent. This specific solvent can be the same solvent used to obtain the crystals described above.

### (Activities and uses)

A crystal of Compound (1) according to an embodiment of the present invention has an excellent EGFR inhibitory activity. In particular, it has an excellent inhibitory activity against EGFR (Del19/C797S), EGFR (L858R/C797S), EGFR (Del19/T790M/C797S), and EGFR (L858R/T790M/C797S), and is useful as an antitumor agent. In addition, a crystal of Compound (1) according to an embodiment of the present invention has excellent selectivity for mutant EGFR and has the advantage of fewer side effects than those caused by wild-type EGFR and other kinases.

Herein, "wild-type EGFR" is represented, for example, by the amino acid sequence of GenBank accession number: NP_005219.2.

"Exon 19" herein refers to the region 729-823 in the amino acid sequence of wild-type EGFR (e.g., GenBank accession number: NP_005219.2).

Herein, "Del19" refers to a mutation in which one or more amino acids are deleted in the exon 19 region of wild-type EGFR. Mutations including, in addition to the deletion in the region, insertion of any one or more amino acids are also included. The mutation of deletion in exon 19 include a mutation in which five amino acids spanning from glutamic acid at position 746 to alanine 750 at position 750 in the exon 19 region are deleted (Del E746-A750 (also referred to as d746-750)), a mutation in which deletion of seven amino acids spanning from leucine at position 747 to proline at position 753 in the exon 19 region is followed by insertion of serine (Del L747-P753insS), a mutation in which five amino acids spanning from leucine at position 747 to threonine at position 751 in the exon 19 region are deleted (Del L747-T751), and a mutation in which deletion of four amino acids spanning from leucine at position 747 to alanine at position 750 in the exon 19 region is followed by insertion of proline (Del L747-A750insP), etc. Preferably, it may be a mutation in which five amino acids spanning from glutamic acid at position 746 to alanine at position 750 in the exon 19 region are deleted (Del E746-A750).

The crystal of the present invention may be used in postoperative adjuvant chemotherapy that is given after surgical resection of a tumor to prevent recurrence, or in preoperative adjuvant chemotherapy given before surgical resection of a tumor.

While the tumor targeted by the present invention is not particularly limited, examples thereof include head and neck cancer, gastrointestinal cancer (esophageal cancer, stomach cancer, duodenal cancer, liver cancer, biliary tract cancer (gallbladder cancer, bile duct cancer, etc.), pancreatic cancer, large bowel cancer (colorectal cancer, colon cancer, rectal cancer, anal cancer, etc.), etc.), lung cancer (non-small cell lung cancer, small cell lung cancer, mesothelioma (pleural mesothelioma, peritoneal mesothelioma, pericardial mesothelioma, testicular mesothelioma, etc.)), breast cancer, genital cancer (ovarian cancer, vulvar cancer, uterine cancer (uterine cervical cancer, uterine body cancer, endometrial cancer, etc.), etc.), urogenital cancer (renal cancer, bladder cancer, prostate cancer, testicular tumor, urothelial cancer, renal pelvis cancer, urethral cancer, etc.), hematopoietic tumor (leukemia, malignant lymphoma, multiple myeloma, etc.), bone and soft tissue tumors, rhabdomyosarcoma, skin cancer, brain tumor, malignant schwannoma, neuroendocrine tumor, thyroid cancer, etc. Preferably, it is head and neck cancer, breast cancer, large bowel cancer, esophageal cancer, pancreatic cancer, lung cancer, ovarian cancer, renal cancer, bladder cancer, skin cancer, or brain tumor, and particularly preferably it is lung cancer. Here, cancers include not only their primary foci but also cancers that have metastasized to other organs (e.g., liver). Furthermore, a crystal of Compound (1) according to an embodiment of the present invention has an excellent inhibitory activity against mutant EGFR. Examples of such mutant EGFR include drug-resistant mutant EGFR and highly sensitive mutant EGFR. Therefore, the compound or a salt thereof of the present invention is also useful as an antitumor agent against the aforementioned malignant tumors with mutant EGFR.

As used herein, the term "effective amount" of a compound means the amount (therapeutically effective amount) of the compound of the present invention, which can cause a biological or medical response such as reduction or inhibition of enzyme and/or protein activity in a subject, which can ameliorate symptoms, alleviate conditions, slow or delay the progress of a disease, or the like.

As used herein, the term "subject" encompasses mammals and non-mammals. According to an embodiment, the subject is a human, and may be a human who has been diagnosed as being in need of the treatment of a symptom, condition, or disease disclosed herein.

Crystals of Compound (1) or salts thereof, or co-crystals thereof can be used as medicines in various dosage forms depending on the purpose of treatment, with or without pulverizing the crystals, and can be used in formulations commonly used for pharmaceutical purposes. The dosage form may be, for example, either an oral dosage form such as a tablet, a capsule, granules, fine granules, powder, or a dry syrup; or a parenteral dosage form such as a suppository, an inhalant, a nasal spray, an ointment, a plaster, or an injection. Pharmaceutical compositions suitable for these dosage forms can be produced by formulation methods known and customary to those skilled in the art, using pharmacologically acceptable carriers.

An embodiment of the present invention provides an antitumor agent for oral administration comprising the above-described crystal of Compound (1) (i.e., crystal of Compound (1) in free form or crystal of Compound (1) with an acid (crystal of salt or co-crystal)). An embodiment of the present invention also provides a method for treating a tumor, the method comprising a step of orally administering an effective amount of the above-described crystal of Compound (1) (i.e., crystal of Compound (1) in free form or crystal of Compound (1) with an acid (crystal of salt or co-crystal)) to a subject in need thereof. Moreover, an embodiment of the present invention provides use of the above-described crystal of Compound (1) (i.e., crystal of Compound (1) in free form or crystal of Compound (1) with an acid (crystal of salt or co-crystal)) for producing an antitumor agent for oral administration. Furthermore, an embodiment of the present invention provides the above-described crystal of Compound (1) (i.e., crystal of Compound (1) in free form or crystal of Compound (1) with an acid (crystal of salt or co-crystal)) for use in the treatment of a tumor by oral administration.

An embodiment of the present invention provides a pharmaceutical composition comprising the above-described crystal of Compound (1) (i.e., crystal of Compound (1) in free form or crystal of Compound (1) with an acid (crystal of salt or co-crystal)). A pharmaceutical composition according to an embodiment of the present invention comprises the above-described crystal of Compound (1) (i.e., crystal of Compound (1) in free form or crystal of Compound (1) with an acid (crystal of salt or co-crystal)) and a pharmacologically acceptable carrier. Moreover, an embodiment of the present invention provides use of the above-described crystal of Compound (1) (i.e., crystal of Compound (1) in free form or crystal of Compound (1) with an acid (crystal of salt or co-crystal)) for producing a pharmaceutical composition. Another embodiment of the present invention provides the above-described crystal of Compound (1) (i.e., crystal of Compound (1) in free form or crystal of Compound (1) with an acid (crystal of salt or co-crystal)) for use as a medicine.

Various organic and inorganic carrier substances customarily used as formulation ingredients can be used as the pharmacologically acceptable carrier, and examples of them that can be blended include an excipient, a binder, a disintegrant, a lubricant, and a coating agent in solid formulations, and a solvent, a solubilizer, a suspending agent, an isotonic agent, a buffering agent, and a soothing agent in liquid formulations. In addition, an antiseptic, an antioxidant, a colorant, a sweetener, a stabilizer, and other formulation additive may also be used if necessary.

Examples of the excipient include starches, saccharides, polysaccharides, and inorganic compounds. Examples of starches include potato starch, corn starch, rice starch, and partially pregelatinized starch. Examples of saccharides include monosaccharides, disaccharides, trisaccharides, and sugar alcohols, such as lactose, white sugar, trehalose, D-mannitol, raffinose, xylitol, and erythritol. Examples of polysaccharides include cellulose and dextran, such as crystalline cellulose, hydroxypropyl cellulose, and hydroxypropyl methyl cellulose. Examples of inorganic compounds include silicates, such as light anhydrous silicic acid and calcium silicate.

Examples of the binder include hydroxypropyl cellulose, methylcellulose, polyvinylpyrrolidone, polyvinyl alcohol, syrup powder, and hypromellose.

Examples of the disintegrant include sodium starch glycolate, calcium carmellose, sodium croscarmellose, crospovidone, low-substituted hydroxypropyl cellulose, and partially pregelatinized starch.

Examples of the lubricant include talc, magnesium stearate, sucrose fatty acid ester, stearic acid, and sodium stearyl fumarate.

Examples of the coating agent include ethyl cellulose, aminoalkyl methacrylate copolymer RS, hypromellose, and white sugar.

Examples of the solvent include water, propylene glycol, and physiological saline solution.

Examples of the solubilizer include polyethylene glycol, alcohols such as ethanol, cyclodextrins, cyclodextrin derivatives, ionic surfactants, and nonionic surfactants, for example, sorbitan fatty acid esters such as polysorbate 80, sucrose fatty acid ester, and sodium lauryl sulfate.

Examples of the suspending agent include carrageenan, crystalline cellulose·sodium carmellose, polyoxyethylene hydrogenated castor oil, gum arabic, and sodium alginate.

Examples of the isotonic agent include sodium chloride, glycerol, and potassium chloride.

Examples of the pH adjuster and the buffering agent include sodium citrate, hydrochloric acid, lactic acid, phosphoric acid, and sodium dihydrogen phosphate.

Examples of the soothing agent include procaine hydrochloride and lidocaine.

Examples of the antiseptic include ethyl p-hydroxybenzoate, cresol, and benzalkonium chloride.

Examples of the antioxidant include sodium sulfite, ascorbic acid, and natural vitamin E.

Examples of the colorant include titanium oxide, iron sesquioxide, edible blue No. 1, and copper chlorophyll.

Examples of the flavoring agent include aspartame, saccharin, sucralose, 1-menthol, and mint flavor.

Examples of the stabilizing agent include sodium pyrosulfite, sodium edetate, erythorbic acid, magnesium oxide, and dibutyl hydroxytoluene.

In the case of preparing an oral solid formulation, the crystal of Compound (1) (i.e., crystal of Compound (1) in free form or crystal of Compound (1) with an acid (crystal of salt or co-crystal)) may be mixed with an excipient and optionally with a binder, a disintegrant, a lubricant, a colorant, a flavoring agent, etc., and then formulated into tablets, coated tablets, granules, powder, capsules, etc. by a conventional method.

In the case of preparing an injection, the crystal of Compound (1) (i.e., crystal of Compound (1) in free form or crystal of Compound (1) with an acid (crystal of salt or co-crystal)) may be mixed with a pH adjuster, a buffering agent, a stabilizer, an isotonic agent, a local anesthetic, etc., and then formulated into injections for subcutaneous, intramuscular, and intravenous use by a conventional method.

The amount of the crystal of Compound (1) (i.e., crystal of Compound (1) in free form or crystal of Compound (1) with an acid (crystal of salt or co-crystal)) to be incorporated into each dosage unit form will vary depending on the symptom of the patient to whom it is applied, or on its formulation, etc. In general, the amount per dosage unit form in terms of Compound (1) in free form is desirably about 0.05 to 1,000 mg for oral formulations, about 0.1 to 500 mg for injections, and about 1 to 1,000 mg for suppositories or topical formulations.

Moreover, the daily dose of the crystal of Compound (1) (i.e., crystal of Compound (1) in free form or crystal of Compound (1) with an acid (crystal of salt or co-crystal)) in the above dosage form will vary depending on the symptom, body weight, age, sex, etc. of the patient. In general, the daily dose for adults (body weight: 50 kg) may usually be about 0.05 to 5,000 mg, and preferably 0.1 to 1,000 mg in terms of Compound (1) in free form.

### EXAMPLES

Hereinafter, the present invention will be further described in detail by means of examples, but the invention is not limited in any way by these examples. Although the invention is sufficiently described by the examples, it is understood that various changes and/or modifications may be made by those skilled in the art. Therefore, as long as such changes and/or modifications do not depart from the scope of the invention, they are encompassed by in the present invention.

In the following examples explaining compounds, % denotes weight percent unless otherwise noted.

### Powder X-ray diffraction measurement

Powder X-ray diffraction was measured under one of the following test conditions, after lightly pulverizing a suitable amount of test substance in an agate mortar as required.

Instrument: EMPYREAN manufactured by PANalytical (Method A)
Reflection method (focusing method)
Target: Cu
X-ray tube current: 40 mA
X-ray tube voltage: 45 kV
Scanned range: 2θ = 5.0 to 40.0°.
Step: 2θ = 0.0131°
Average time/step: 8.670 s
Scan speed: 0.0015°/s
Divergence slit: 1°
Scattering slit: 2.0 mm
Receiving slit: 8.0 mm
Instrument: EMPYREAN manufactured by PANalytical (Method B)
Transmission method
Target: Cu
X-ray tube current: 40 mA
X-ray tube voltage: 45 kV
Scanned range: 2θ = 2.0 to 40.0°
Step: 2θ = 0.0066°
Average time/step: 8.670 s
Scan speed: 0.0008°/s
Divergence slit: 1/2°
Scattering slit: 2.0 mm
Receiving slit: None

The instrument, including data processing, was handled in accordance with the method and procedure indicated for each instrument.

The values obtained from various spectra may somewhat vary depending on the direction of crystal growth, particle size, measurement conditions, and so on. Therefore, these values should not be strictly interpreted.

Simultaneous thermogravimetric-differential thermal analysis (TG-DTA) was performed on 1 to 5 mg of the test substance under the following test conditions.
Instrument: TG/DTA7200
manufactured by Hitachi High-Tech Science Corporation
Sample vessel: Made of aluminum
Heating rate: Temperature was increased from 25°C to 400°C at 10°C/min.
Atmosphere gas: Atmospheric air (200 mL/min)
Control substance: Empty pan

The instrument, including data processing, was handled in accordance with the method and procedure indicated for each instrument.

### Example 1: Production of type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1))

### (Step 1) Synthesis of tert-butyl(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)carbamate

Tert-butyl(4-(4-amino-6-bromo-5-(quinolin-3-yl)-7H-pyrrolo[2,3-d]pyrimidin-7-yl)bicyclo[2.2.1]heptan-1-yl)carbamate (65.9 g) synthesized according to the method described in Patent Literature 1 was dissolved in N-methylpyrrolidone (1,500 mL) by stirring at 90°C for 30 minutes in a nitrogen atmosphere. After cooling to 35°C, diisopropylethylamine (46.5 g), triethylsilylacetylene (33.7 g), copper(I) iodide (4.57 g), dichlorobis[di-tert-butyl(4-dimethylaminophenyl)phosphine]palladium(II) (8.50 g), and N-methylpyrrolidone (80 mL) were added, and the mixture was stirred at 90°C for 2 hours in a nitrogen atmosphere. After cooling to 40°C, 10% aqueous sodium dihydrogen phosphate solution and ethyl acetate were added. The organic layer was washed with diluted ammonia and water, to which activated carbon (33.0 g) and SH silica gel (65.9 g) were added and the mixture was stirred at 40°C for 1 hour and at room temperature for 2 hours. The insoluble substance was filtered over Celite, and then solvent was removed under reduced pressure. Acetonitrile was added to the residue and the mixture was stirred at room temperature for 15 hours. The precipitated solid was filtered, washed with acetonitrile, and dried under reduced pressure to give the title compound (37.4 g).

### (Step 2) Synthesis of 7-(4-aminobicyclo[2.2.1]heptan-1-yl)-5-(quinolin-3-yl)-6-((triethylsilyl)ethynyl)-7H-pyrrolo[2,3-d]pyrimidin-4-amine

To tert-butyl(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)carbamate (65.0 g), 5-10% hydrochloric acid methanol solution (520 mL) was added and the mixture was stirred at 50°C for 3 hours in a nitrogen atmosphere. Under ice-cold conditions, 2 mol/L sodium hydroxide solution was added dropwise to bring the pH to around 12. The precipitated solid was filtered, washed with 50% methanol, and dried under reduced pressure to give the title compound (50.3 g).

### (Step 3) Synthesis of N-(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)-5-methylpyrazine-2-carboxamide

To a mixture of 7-(4-aminobicyclo[2.2.1]heptan-1-yl)-5-(quinolin-3-yl)-6-((triethylsilyl)ethynyl)-7H-pyrrolo[2,3-d]pyrimidin-4-amine (48.3 g) obtained in Step 2, 5-methylpyrazine-2-carboxylic acid (13.4 g), 1-hydroxybenzotriazole monohydrate (16.0 g), diisopropylethylamine (24.6 g), and methylene chloride (1,450 mL), 1-(3-dimethylaminopropyl)-3-ethylcarbodiimide hydrochloride (21.9 g) was added in a nitrogen atmosphere and the mixture was stirred for 17 hours. The reaction solution was washed with 10% aqueous sodium dihydrogen phosphate solution, saturated aqueous sodium carbonate solution, and saturated saline solution, and dried over anhydrous sodium sulfate. The drying agent was filtered off and the solvent was removed under reduced pressure. To the obtained residue, methanol was added and the mixture was stirred at room temperature for 2 hours. The precipitated solid was filtered, washed with methanol, and dried under reduced pressure to give the title compound (54.8 g).

### (Step 4) Synthesis of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1))

To a mixture of N-(4-(4-amino-5-(quinolin-3-yl)-6-((triethylsilyl)ethynyl)-7H-pyrrolo[2,3-d]pyrimidin-7-yl)bicyclo[2.2.1]heptan-1-yl)-5-methylpyrazine-2-carboxamide (80.0 g), methylene chloride (1,000 mL), and methanol (1,000 mL), potassium carbonate (7.03 g) was added in a nitrogen atmosphere and the mixture was stirred for 18 hours. The insoluble substance was filtered over Celite, and the solvent was removed under reduced pressure. To the obtained residue, methanol and water were added and the mixture was stirred for 1 hour. The precipitated solid was filtered, washed with 50% methanol, and dried under reduced pressure to give the title compound (64.5 g) as a pale yellow solid (type III crystal). The structure of the obtained pale yellow solid was analyzed by 1H-NMR (instrument: AVNEO 400 manufactured by Bruker, 400 MHz, Scans: 4, DMSO-d6) and confirmed to be N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1)).

The powder X-ray diffraction spectrum (method A) of the type III crystal obtained in Step 4 was acquired by the procedure described above and is shown in Figure 1. The characteristic diffraction angles observed in the powder X-ray diffraction spectrum included the following peaks.
Characteristic diffraction angles (20 ± 0.2°): 6.6°, 8.8°, 11.6°, 12.3°, 13.8°, 17.9°, 18.6°, 21.3°, and 22.3

Other peaks are shown in Table 1 below.

**Table 1**

| Position of peak (°2θ) | Intensity (cts) |
|---|---|
| 6.6 | 377 |
| 7.9 | 75 |
| 8.8 | 191 |
| 11.6 | 220 |
| 12.3 | 140 |
| 13.2 | 1,026 |
| 13.8 | 3,936 |
| 15.0 | 350 |
| 15.7 | 926 |
| 15.9 | 510 |
| 17.2 | 53 |
| 17.9 | 325 |
| 18.6 | 1,578 |
| 19.7 | 61 |
| 20.2 | 68 |
| 21.3 | 413 |
| 22.3 | 659 |
| 23.0 | 125 |
| 23.6 | 179 |
| 24.1 | 156 |
| 25.6 | 778 |
| 26.0 | 820 |
| 26.6 | 328 |
| 27.0 | 625 |
| 28.3 | 63 |
| 29.1 | 105 |
| 30.7 | 235 |
| 31.6 | 114 |
| 32.2 | 101 |
| 32.9 | 53 |
| 33.5 | 67 |
| 33.8 | 77 |
| 34.6 | 47 |
| 35.2 | 94 |
| 36.6 | 46 |

The simultaneous thermogravimetric-differential thermal analysis curve of the crystal obtained in Step 4 was acquired by the procedure described above and is shown in Figure 2. An exothermic peak (onset value) was observed at around 228°C in the simultaneous thermogravimetric-differential thermal analysis curve.

### Example 2: Production of type II crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1))

A type III crystal of Compound (1) in free form was synthesized in the same manner as in Example 1. The same procedure was employed for other examples, reference example, and comparative examples described below.

Tetrahydrofuran (6 mL) was added to the type III crystal of Compound (1) in free form (300 mg), and the suspension was stirred at 25°C for about 24 hours. The resultant was filtered to collect the solid, which was dried under reduced pressure at room temperature for about 30.5 hours to give 199.3 mg of the title crystal.

The powder X-ray diffraction spectrum (method A) of the resulting crystal was acquired by the procedure described above and is shown in Figure 3.

The characteristic diffraction angles observed in the powder X-ray diffraction spectrum included the following peaks.
Characteristic diffraction angles (2θ ± 0.2°): 7.8°, 10.6°, 14.0°, 14.8°, 15.6°, 17.2°, 20.1°, 21.6°, 22.2°, 24.7°, 25.8°, and 28.9°

Other peaks are shown in Table 2 below.

**Table 2**

| Position of peak (°2θ) | Intensity (cts) |
|---|---|
| 7.8 | 413 |
| 8.6 | 71 |
| 10.6 | 454 |
| 12.4 | 102 |
| 13.1 | 78 |
| 14.0 | 1,012 |
| 14.4 | 675 |
| 14.8 | 2,212 |
| 15.1 | 330 |
| 15.6 | 601 |
| 17.2 | 289 |
| 18.4 | 223 |
| 19.0 | 256 |
| 20.1 | 390 |
| 20.6 | 375 |
| 21.2 | 317 |
| 21.6 | 3,076 |
| 22.2 | 1,525 |
| 22.7 | 79 |
| 23.3 | 107 |
| 24.1 | 240 |
| 24.7 | 648 |
| 25.8 | 578 |
| 26.2 | 273 |
| 26.8 | 101 |
| 27.4 | 452 |
| 27.6 | 273 |
| 28.2 | 160 |
| 28.9 | 1,042 |
| 29.4 | 251 |
| 29.8 | 126 |
| 30.4 | 121 |
| 30.9 | 272 |
| 31.9 | 199 |
| 32.7 | 130 |
| 33.4 | 69 |
| 34.1 | 98 |
| 34.7 | 118 |
| 35.7 | 64 |
| 37.4 | 58 |
| 38.3 | 78 |
| 38.9 | 60 |
| 39.4 | 51 |

The simultaneous thermogravimetric-differential thermal analysis curve of the resulting crystal was acquired by the procedure described above and is shown in Figure 4. An exothermic peak (onset value) was observed at around 236°C in the simultaneous thermogravimetric-differential thermal analysis curve.

### Reference example 1: Production of type VII crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1))

A mixture of water and ethanol (1:1 v/v) (6 mL) was added to the type III crystal of Compound (1) in free form (300 mg). The suspension was stirred at 50°C for about 2 hours, and then filtered to collect the solid, thereby obtaining type VII crystal in free form. The resultant was dried under reduced pressure at room temperature for about 65 hours to give 286.9 mg of the title crystal.

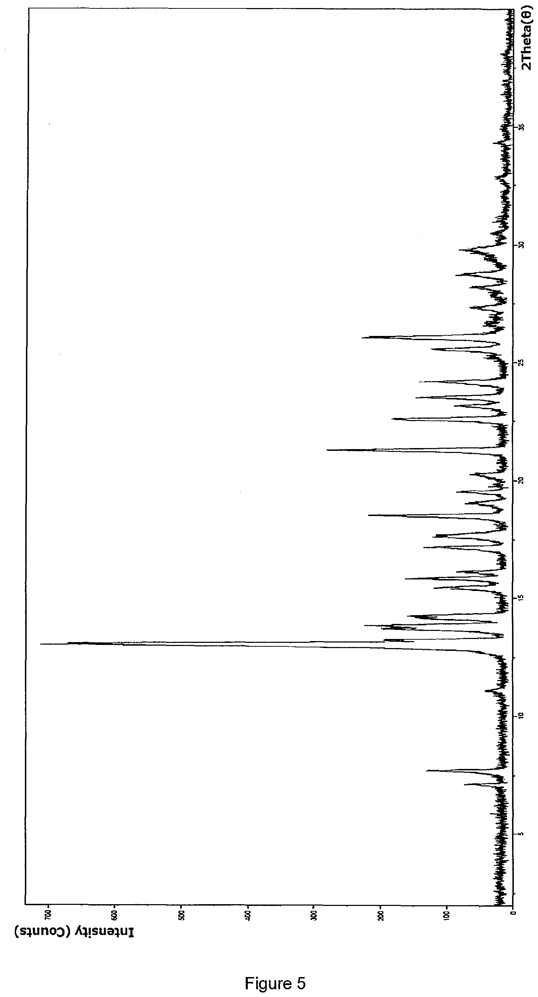

The powder X-ray diffraction spectrum (method B) of the resulting crystal was acquired by the procedure described above and is shown in Figure 5. The characteristic diffraction angles observed in the powder X-ray diffraction spectrum included the following peaks.

Characteristic diffraction angles (2θ ± 0.2°): 7.1°, 7.7°, 13.0°, 17.2°, 18.5°, 21.3°, 22.6°, 23.5°, 24.2°, and 26.1°

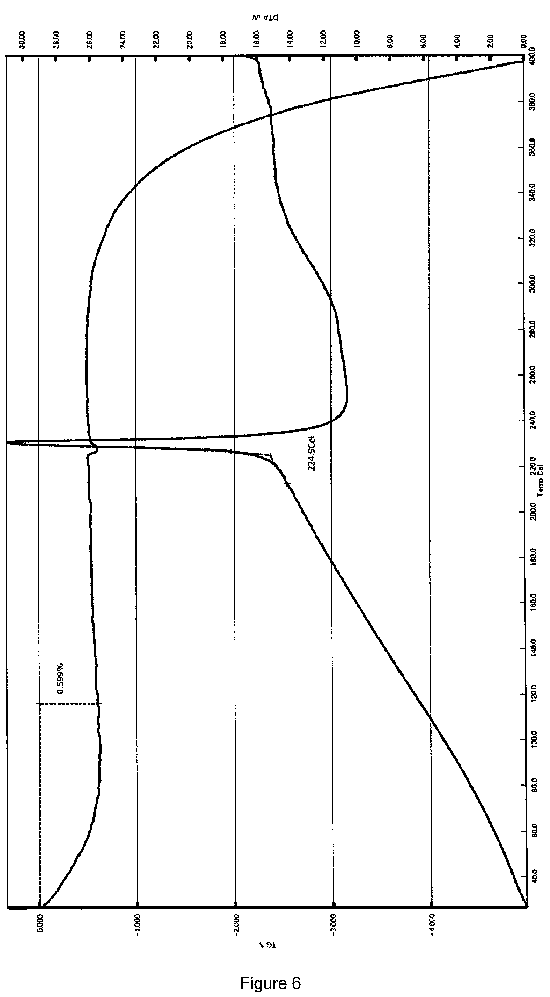

The simultaneous thermogravimetric-differential thermal analysis curve of the resulting crystal was acquired by the procedure described above and is shown in Figure 6. An exothermic peak (onset value) was observed at around 225°C in the simultaneous thermogravimetric-differential thermal analysis curve.

### Comparative example 1: Production of type VIII crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1))

The type III crystal of Compound (1) in free form (10.0 g) was dissolved in dimethyl sulfoxide (140 mL) at 45°C, and cooled to 30-35°C. Then, dimethyl sulfoxide (10 mL) and water (37.5 mL) were added, and the mixture was stirred at room temperature for 5.5 hours and filtered to collect the solid, which was dried under reduced pressure at 50°C to give 9.6 g of the title crystal.

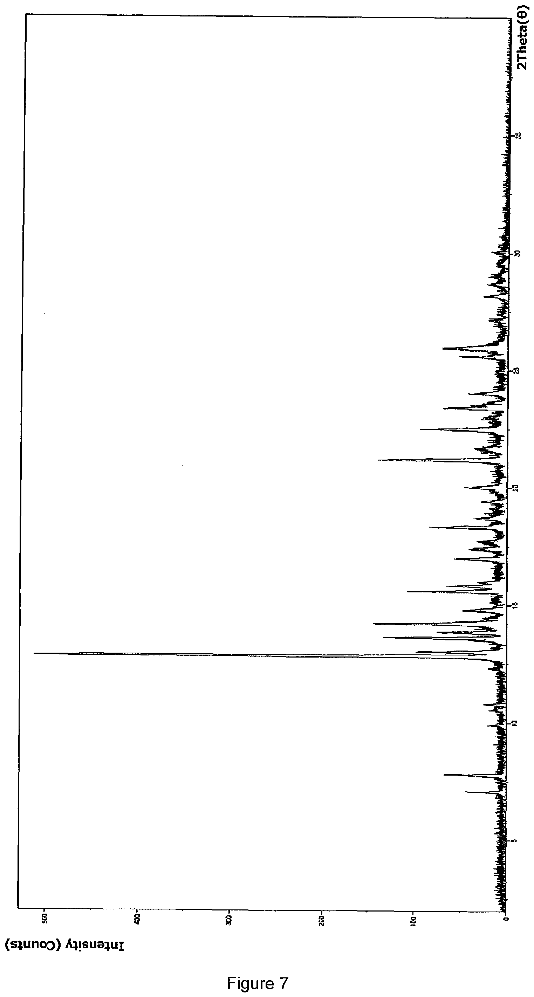

The powder X-ray diffraction spectrum (method B) of the resulting crystal was acquired by the procedure described above and is shown in Figure 7. The characteristic diffraction angles observed in the powder X-ray diffraction spectrum were as follows.
Characteristic diffraction angles (20 ± 0.2°): 7.1°, 7.8°, 12.8°, 13.6°, 14.2°, 14.8°, 17.0°, 18.3°, 21.2°, 22.5°, 24.0°, and 25.9°

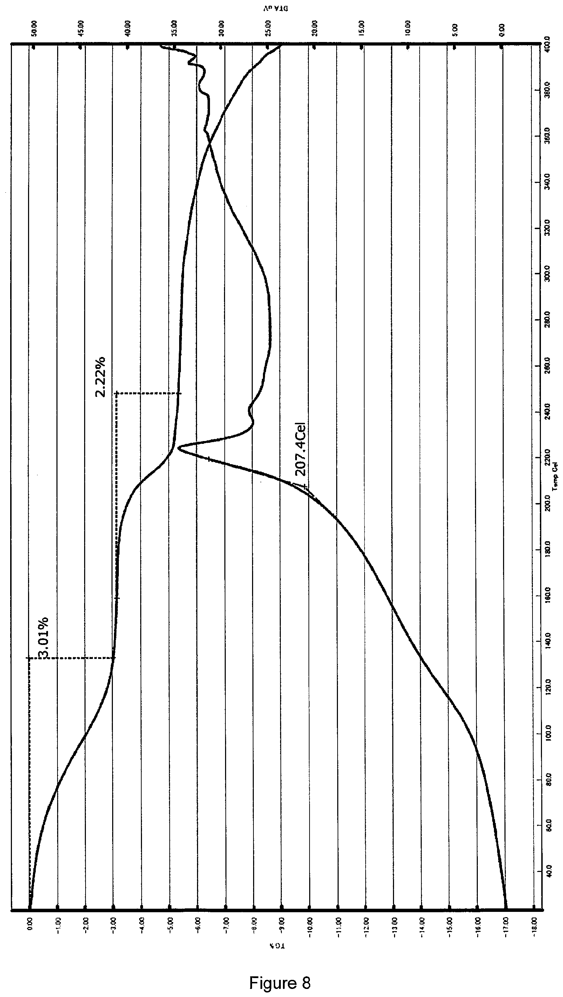

The simultaneous thermogravimetric-differential thermal analysis curve of the resulting crystal was acquired by the procedure described above and is shown in Figure 8. An exothermic peak (onset value) was observed at around 207°C in the simultaneous thermogravimetric-differential thermal analysis curve.

### Test Example 1: Solid stability test

The solid stability of the type II crystal of Compound (1) in free form, the type III crystal of Compound (1) in free form, the type VII crystal of Compound (1) in free form, and the type VIII crystal of Compound (1) in free form obtained in the examples, reference example, and comparative example were evaluated after 4 weeks of storage by the following procedure.
Storage conditions: 80°C (closed system) (HIFLEX (high-temperature storage chamber manufactured by ETAC))
Storage period: 4 weeks
Storage volume: Approximately 30 mg
Storage container: Glass bottle

The results are shown in Table 3 below.

**Table 3**

| | Chemical purity before storage | Chemical purity after 4 weeks of storage at 80°C | Change |
|---|---|---|---|
| Type II crystal in free form | 99.80% | 99.78% | -0.02% |
| Type III crystal in free form | 99.58% | 99.60% | +0.02% |
| Type VII crystal in free form | 99.63% | 99.63% | - |
| Type VIII crystal in free form | 99.62% | 99.43% | -0.19% |

Changes in the mass of the related substances (the amounts of detected substances other than Compound (1)) were analyzed by HPLC according to the following method. Approximately 8 mg of each sample was weighed and dissolved in 1 mL of a chloroform-methanol mixture (1:1 v/v). Fifty µL of this solution was measured and diluted with 950 µL of an acetonitrile-water mixture (7:3), and 5 µL of the resulting solution was accurately measured and used as the sample for the analysis.

### HPLC assay (stability test)

The mass of the related substances in the sample solution was determined by HPLC analysis. The instrument, including data processing, was handled in accordance with the method and procedure indicated for each instrument.
Column: InertSustainSwift C18 (2.1 x 50 mm, 1.9 µm) manufactured by GL Sciences
UV detection: 220 nm
Column temperature: 40°C
Flow rate: 0.3 mL/min
Sample cooler: 10°C
Sample concentration: 0.1 mg/mL
Mobile phase A: 10 mmol/L phosphate buffer (pH 6.8)/acetonitrile (95:5) mixture
Mobile phase B: Acetonitrile

The gradients are shown in Table 4.

**Table 4**

| Time (min.) | Mobile phase A (vol%) | Mobile phase B (vol%) |
|---|---|---|
| 0 | 80 | 20 |
| 18 | 21 | 79 |
| 20 | 21 | 79 |
| 20.5 | 80 | 20 |
| 25 | 80 | 20 |

As a result, the type II crystal of Compound (1) in free form, the type III crystal of Compound (1) in free form, and the type VII crystal of Compound (1) in free form were found to be extremely stable crystals with almost no increase in the related substances.

### Test Example 2: Dynamic vapor sorption (DVS) test

Vapor sorption tests were performed using the type II crystal of Compound (1) in free form, the type III crystal of Compound (1) in free form, the type VII crystal of Compound (1) in free form, and the type VIII crystal of Compound (1) in free form obtained in the examples, reference example, and comparative example.

Vapor sorption tests were conducted under the following conditions.

Approximately 10 mg of the sample was filled into a specialized quartz holder to continuously measure and record the weight of the sample at each humidity was under the following conditions. The instrument, including data processing, was handled in accordance with the method and procedure indicated for each instrument.
Instrument: VTI-SA+ (manufactured by TA Instruments)
Drying temperature: 60°C
Heating rate: 1°C/min
Equilibration of drying: Confirmation of no more than 0.01 wt% decrease in 5 minutes within a period not exceeding 300 minutes
Measurement temperature: 25°C
Equilibration of humidification: Confirmation of no more than 0.01 wt% increase in 5 minutes within a period not exceeding 120 minutes
Relative humidity program: Increase from 5 to 95% RH in increments of 5% RH, and decrease from 95% to 5% RH in increments of 5% RH.

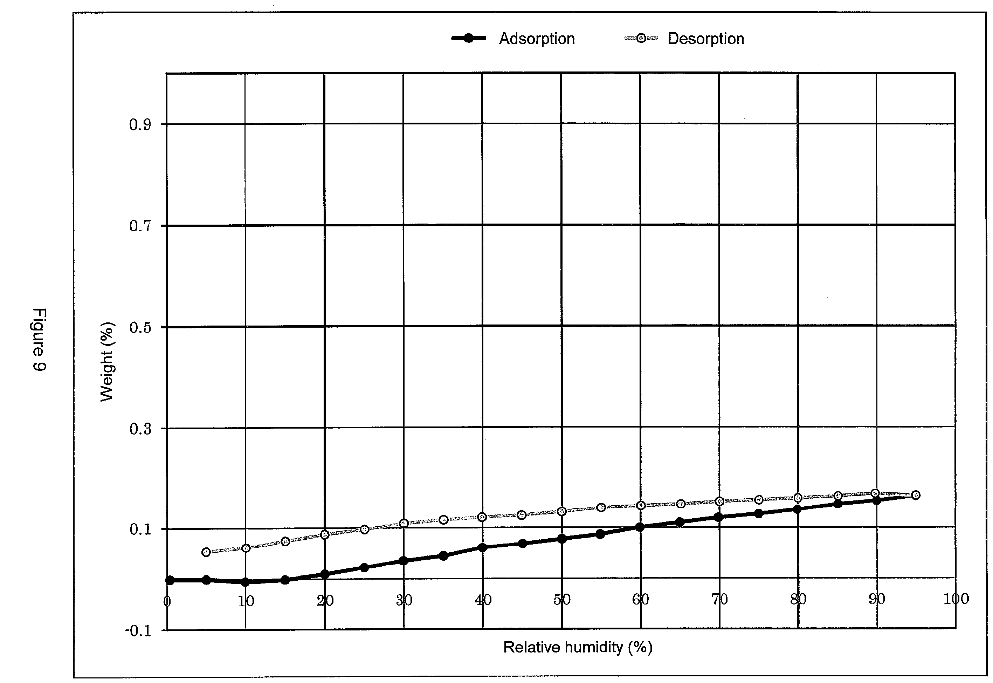

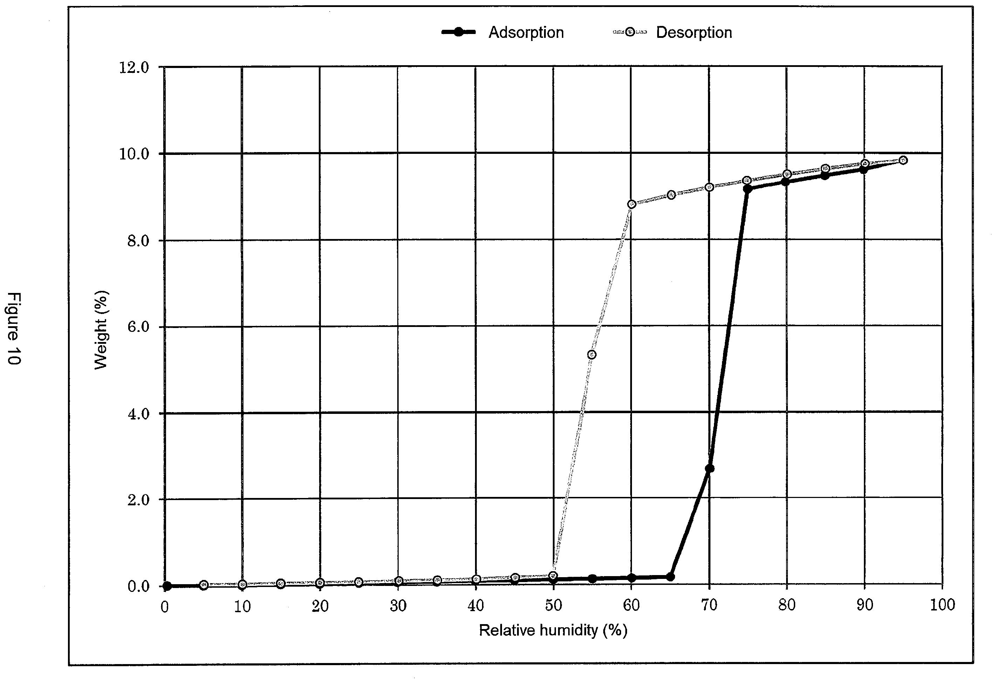

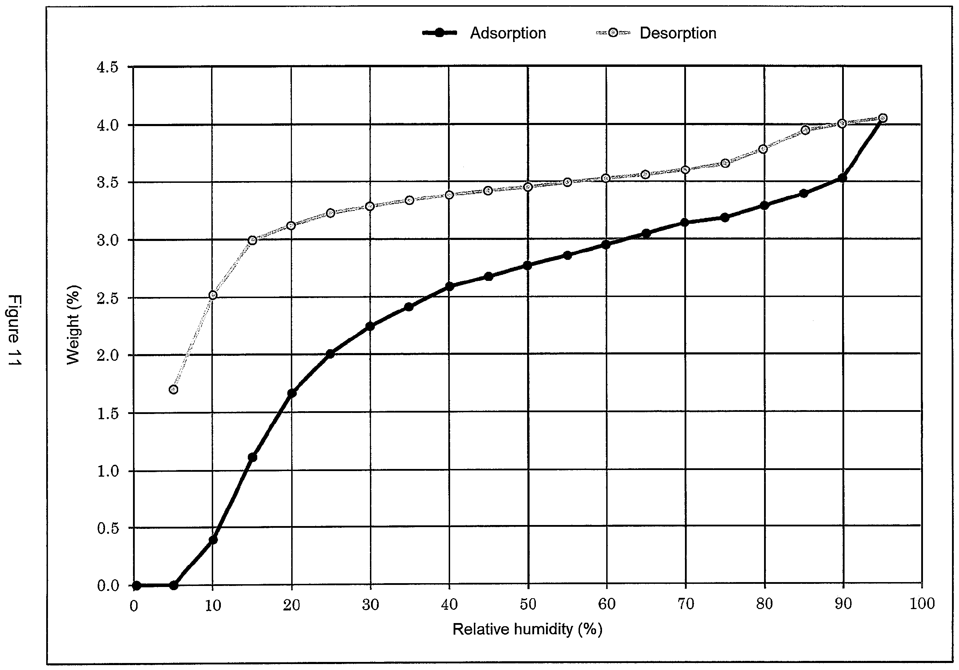

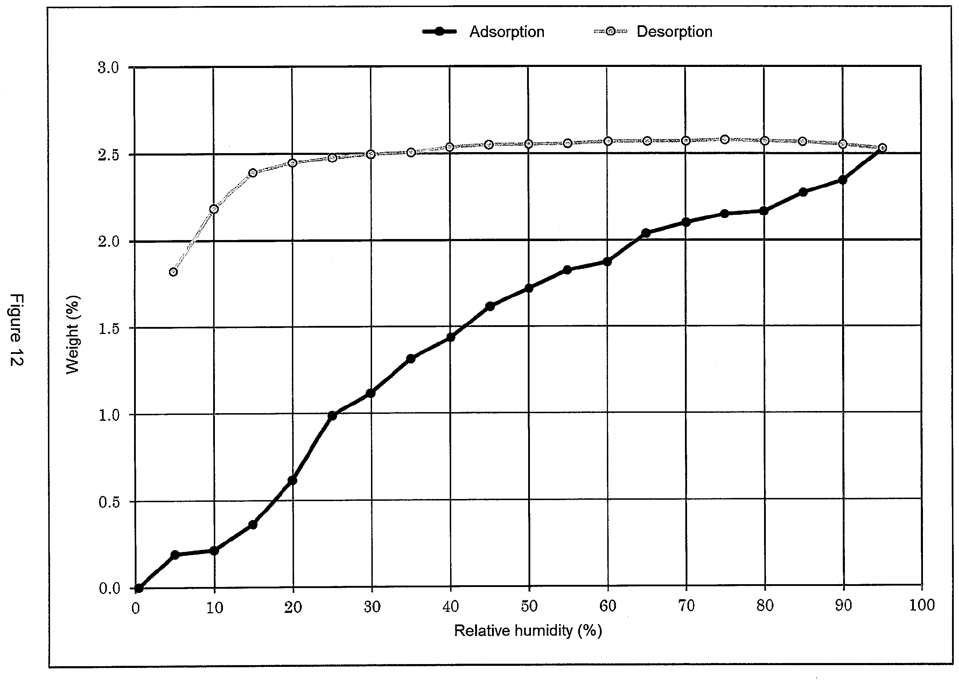

Changes in the weight over the range of the measurement conditions obtained in these tests are shown in Figures 9 through 12.

As shown in the figures, the type II crystal of Compound (1) in free form showed a weight increase of 0.16% under 95% relative humidity in the vapor sorption test, and this weight increase of less than 1% indicates that it was hardly hygroscopic. The type III crystal of Compound (1) in free form showed hygroscopicity that sharply increased at above 65% RH, but this adsorption was found to be reversible since desorption of water occurred when the humidity was decreased. On the other hand, as shown in the figures, the type VII crystal in free form and the type VIII crystal in free form showed mass increases of 1% or more under 95% relative humidity in the vapor sorption test, indicating that they were highly hygroscopic. Furthermore, these crystals did not cause desorption of water even when the humidity was decreased, indicating that the reaction was irreversible.

The above results showed that the type II crystal of Compound (1) in free form had lower hygroscopicity than the type III crystal of Compound (1) in free form, the type VII crystal of Compound (1) in free form, and the type VIII crystal of Compound (1) in free form, and was superior in terms of industrial manufacturing of a pharmaceutical product of stable quality among the candidate compounds for the development of the pharmaceutical product. The type III crystal in free form was considered to be useful, like the type II crystal in free form, as a candidate compound for a pharmaceutical product in controlled humidity environment, because it has low hygroscopicity as long as RH is less than 65%. The type II crystal of Compound (1) in free form and the type III crystal of Compound (1) in free form were confirmed to have excellent properties as pharmaceutical products or active pharmaceutical ingredients.

### Example 3: Production of type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1)) with succinic acid

Succinic acid (6.88 g) and acetonitrile (90 mL) were added to the type III crystal of Compound (1) in free form (10 g), and the suspension was stirred at 50°C for about 1 hour, and then filtered to collect the solid. The solid was suspended/washed twice with ethanol and dried under reduced pressure at 40°C for about 19.5 hours to give 11.71 g of the title crystal.

The powder X-ray diffraction spectrum (method A) of the resulting crystal was acquired by the procedure described above and is shown in Figure 13. The characteristic diffraction angles observed in the powder X-ray diffraction spectrum included the following peaks.

Characteristic diffraction angles (2θ ± 0.2°): 10.4°, 13.3°, 14.6°, 15.7°, 17.2°, 18.3°, 19.7°, 20.8°, 22.3°, and 27.5°

Other peaks are shown in Table 5 below.

**Table 5**

| Position of peak (°2θ) | Intensity (cts) |
|---|---|
| 7.5 | 54 |
| 8.2 | 93 |
| 10.4 | 350 |
| 11.6 | 84 |
| 12.3 | 545 |
| 13.3 | 879 |
| 13.7 | 54 |
| 14.6 | 825 |
| 14.9 | 467 |
| 15.7 | 484 |
| 16.7 | 123 |
| 17.2 | 788 |
| 18.3 | 1,114 |
| 18.6 | 123 |
| 19.7 | 2,557 |
| 20.4 | 248 |
| 20.8 | 372 |
| 21.2 | 53 |
| 21.7 | 106 |
| 22.3 | 491 |
| 22.8 | 78 |
| 23.2 | 368 |
| 23.5 | 334 |
| 24.4 | 491 |
| 24.7 | 784 |
| 25.4 | 110 |
| 26.0 | 358 |
| 27.5 | 469 |
| 28.2 | 59 |
| 28.6 | 79 |
| 29.2 | 168 |
| 29.4 | 202 |
| 29.7 | 215 |
| 30.4 | 174 |
| 31.1 | 269 |
| 31.6 | 130 |
| 32.1 | 28 |
| 32.6 | 51 |
| 33.3 | 176 |
| 33.7 | 56 |
| 34.2 | 123 |
| 34.8 | 81 |
| 35.2 | 125 |
| 36.7 | 65 |
| 37.6 | 56 |
| 38.1 | 54 |

The simultaneous thermogravimetric-differential thermal analysis curve of the resulting crystal was acquired by the procedure described above and is shown in Figure 14. An endothermic peak (onset value) was observed at around 202°C in the simultaneous thermogravimetric-differential thermal analysis curve.

### Example 4: Production of type I crystal of N-(4-(4-amino-6-ethynyl-5-(quinolin-3-yl)-7H-pyrrolo[2,3-dipyrimidin-7-yl)bicyclo[2.2.1]heptan-1-yl)-5-methylpyrazine-2-carboxamide (Compound (1)) with sorbic acid

Sorbic acid (109 mg) and acetonitrile (5 mL) were added to the type III crystal of Compound (1) in free form (500 mg). The suspension was stirred at 50°C for about 7.5 hours, and then filtrated to collect the solid, which was dried under reduced pressure at 40°C for about 13.5 hours to give 568.4 mg of the title crystal.

The powder X-ray diffraction spectrum (method A) of the resulting crystal was acquired by the procedure described above and is shown in Figure 15.

The characteristic diffraction angles observed in the powder X-ray diffraction spectrum included the following peaks.

Characteristic diffraction angles (2θ ± 0.2°): 12.6°, 14.7°, 15.4°, 15.9°, 17.1°, 18.9°, 22.2°, 24.1°, 25.8°, and 27.4°

Other peaks are shown in Table 6 below.

**Table 6**

| Position of peak (°2θ) | Intensity (cts) |
|---|---|
| 5.2 | 267 |
| 7.5 | 48 |
| 9.1 | 105 |
| 10.3 | 84 |
| 12.3 | 629 |
| 12.6 | 1,011 |
| 13.7 | 88 |
| 14.3 | 109 |
| 14.7 | 525 |
| 15.4 | 860 |
| 15.9 | 807 |
| 16.2 | 163 |
| 17.1 | 858 |
| 17.6 | 257 |
| 18.9 | 1,892 |
| 19.6 | 167 |
| 20.6 | 118 |
| 21.6 | 605 |
| 22.2 | 1,384 |
| 22.5 | 144 |
| 22.9 | 87 |
| 24.1 | 329 |
| 24.7 | 98 |
| 25.2 | 157 |
| 25.8 | 1,157 |
| 26.3 | 82 |
| 26.7 | 144 |
| 27.4 | 707 |
| 28.0 | 162 |
| 28.3 | 152 |
| 28.9 | 493 |
| 29.4 | 208 |
| 29.9 | 154 |
| 30.5 | 71 |
| 30.8 | 85 |
| 31.5 | 78 |
| 32.2 | 32 |
| 32.8 | 116 |
| 34.1 | 85 |
| 34.9 | 63 |
| 36.0 | 77 |
| 36.7 | 62 |
| 37.1 | 50 |
| 37.4 | 52 |
| 38.5 | 105 |
| 38.9 | 126 |
| 39.5 | 28 |

The simultaneous thermogravimetric-differential thermal analysis curve of the resulting crystal was acquired by the procedure described above and is shown in Figure 16. An endothermic peak (onset value) was observed at around 173°C in the simultaneous thermogravimetric-differential thermal analysis curve.

### Comparative example 2: Production of type I crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1)) with citric acid

Citric acid (560 mg) and 10 mL of ethanol were added to the type III crystal of Compound (1) in free form (500 mg). The suspension was stirred at 25°C for about 5.5 hours, and then filtrated to collect the solid, which was dried under reduced pressure at 40°C for about 13.5 hours to give 664.3 mg of the title crystal.

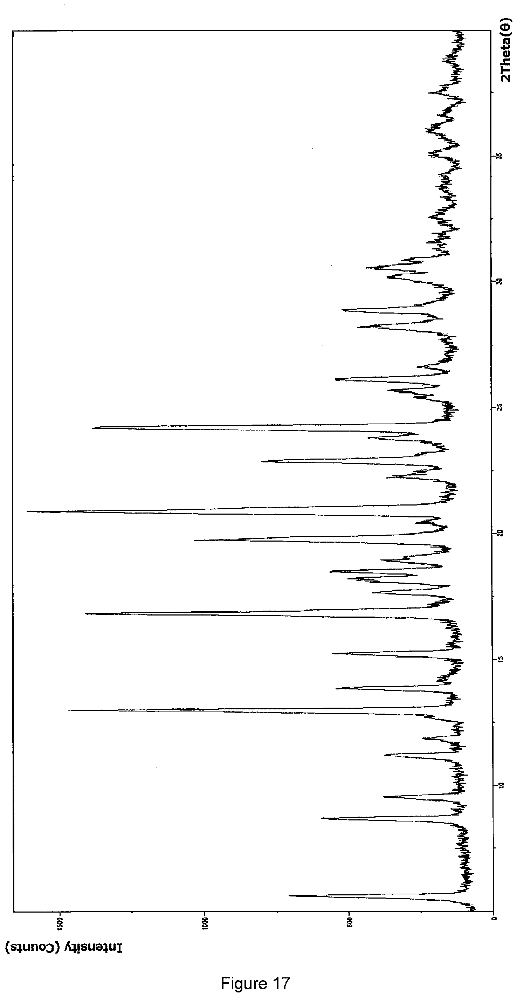

The powder X-ray diffraction spectrum (method A) of the resulting crystal was acquired by the procedure described above and is shown in Figure 17. The characteristic diffraction angles observed in the powder X-ray diffraction spectrum included the following peaks.

Characteristic diffraction angles (2θ ± 0.2°): 5.6°, 8.7°, 9.6°, 11.2°, 13.0°, 13.9°, 15.2°, 16.8°, 20.9°, and 24.2°

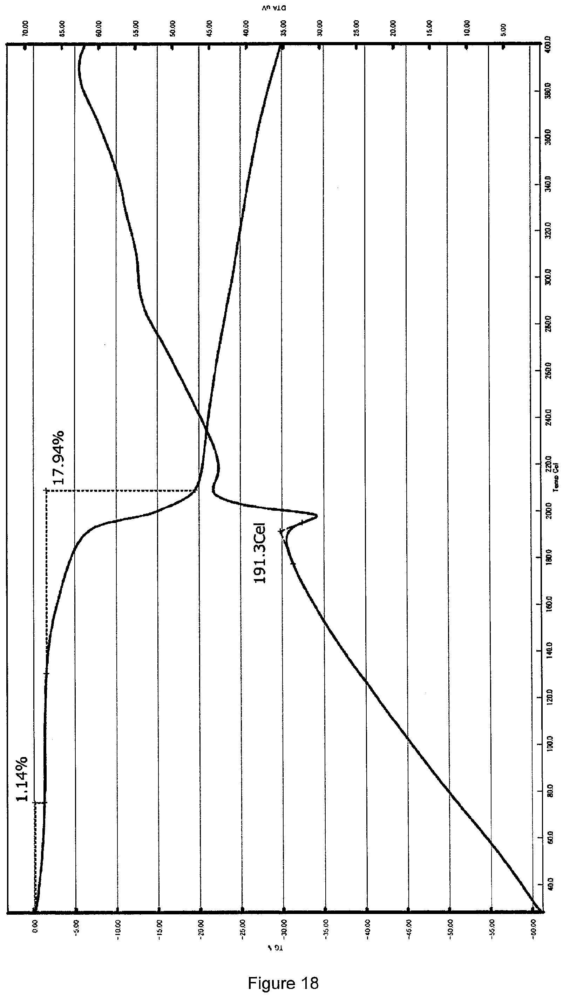

The simultaneous thermogravimetric-differential thermal analysis curve of the resulting crystal was acquired by the procedure described above and is shown in Figure 18. An endothermic peak (onset value) was observed at around 191°C in the simultaneous thermogravimetric-differential thermal analysis curve.

### Comparative example 3: Production of type IV crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1)) with citric acid

Citric acid (37.3 mg) and tetrahydrofuran (3 mL) were added to the type III crystal of Compound (1) in free form (100 mg). The suspension was stirred at 50°C for about 47.5 hours, and then filtrated to collect the solid, which was dried under reduced pressure at 40°C for about 18.5 hours to give 85.6 mg of the title crystal.

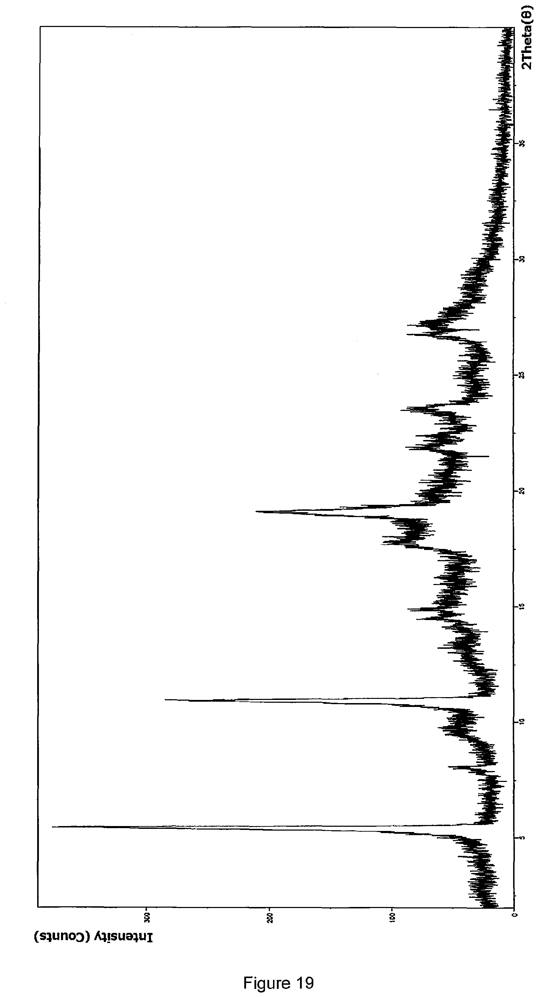

The powder X-ray diffraction spectrum (method B) of the resulting crystal was acquired by the procedure described above and is shown in Figure 19. The characteristic diffraction angles observed in the powder X-ray diffraction spectrum included the following peaks.
Characteristic diffraction angles (2θ ± 0.2°): 5.4°, 11.0°, and 19.1°

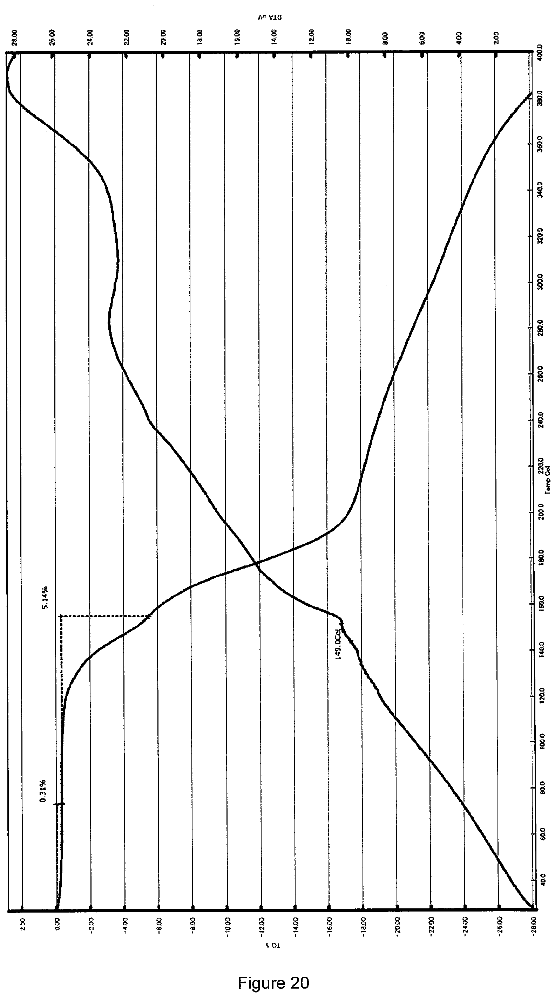

The simultaneous thermogravimetric-differential thermal analysis curve of the resulting crystal was acquired by the procedure described above and is shown in Figure 20. An endothermic peak (onset value) was observed at around 149°C in the simultaneous thermogravimetric-differential thermal analysis curve.

### Comparative example 4: Production of type I crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide (Compound (1)) with phosphoric acid

Phosphoric acid (39.7 µL) and tetrahydrofuran (6 mL) were added to the type III crystal of Compound (1) in free form (300 mg). The suspension was stirred at 50°C for about 3 hours, and then filtrated to collect the solid, which was dried under reduced pressure at room temperature for about 40 minutes to give 111.6 mg of the title crystal.

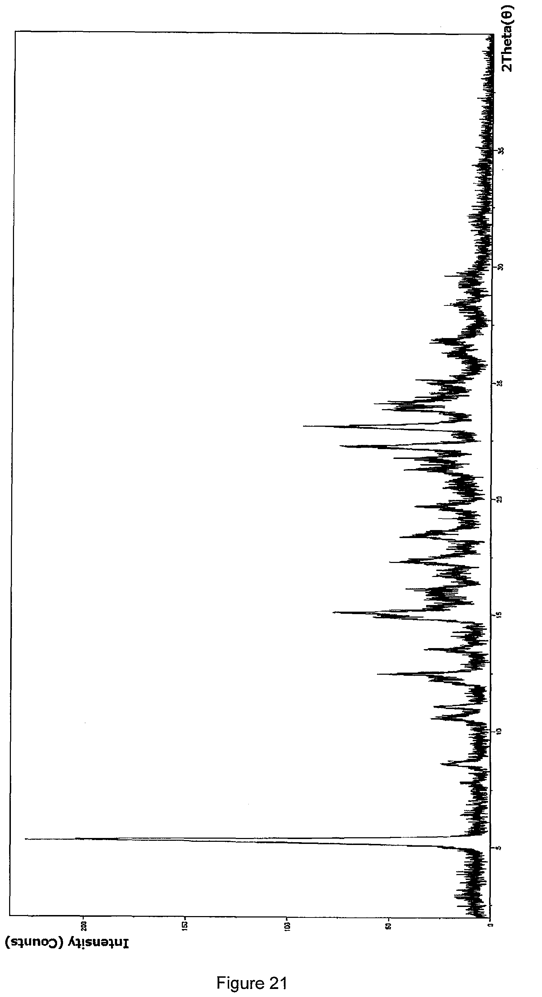

The powder X-ray diffraction spectrum (method B) of the resulting crystal was acquired by the procedure described above and is shown in Figure 21. The characteristic diffraction angles observed in the powder X-ray diffraction spectrum included the following peaks.

Characteristic diffraction angles (2θ ± 0.2°): 5.3°, 8.6°, 10.6°, 11.1°, 12.5°, 13.5°, 17.3°, 19.7°, 22.3°, and 23.1°

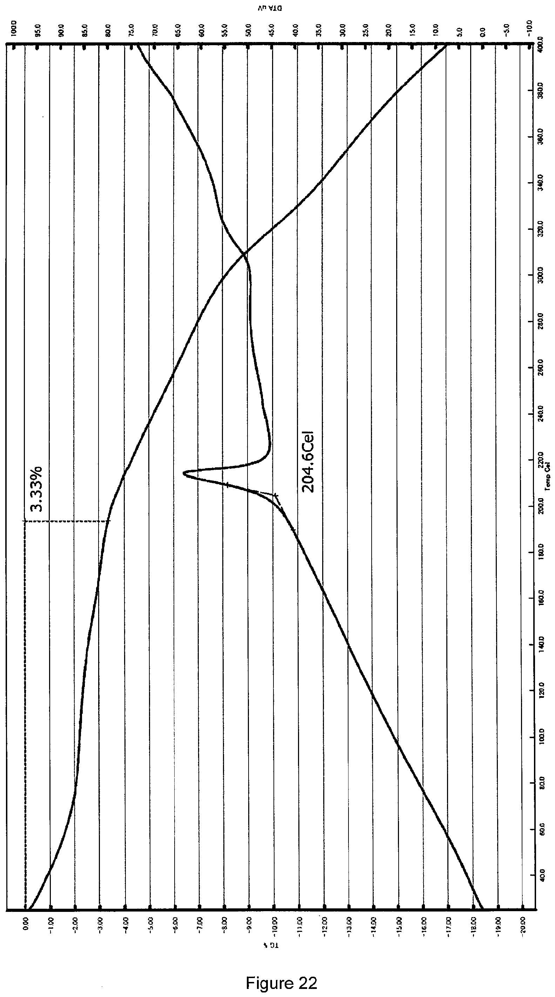

The simultaneous thermogravimetric-differential thermal analysis curve of the resulting crystal was acquired by the procedure described above and is shown in Figure 22. An exothermic peak (onset value) was observed at around 205°C in the simultaneous thermogravimetric-differential thermal analysis curve.

### Test Example 3: Solid stability test

The solid stability of the type III crystal of Compound (1) with succinic acid, the type I crystal of Compound (1) with citric acid, the type IV crystal of Compound (1) with citric acid, the type I crystal of Compound (1) with sorbic acid, and the type I crystal of Compound (1) with phosphoric acid obtained in the examples, reference example, and comparative examples were evaluated after 4 weeks of storage. The evaluation method, storage, and measurement conditions were as described in Test Example 1.

The results are shown in Table 7 below.

**Table 7**

| | Chemical purity before storage | Chemical purity after 4 weeks of storage at 80°C | Change |
|---|---|---|---|
| Type III crystal of Compound (1) with succinic acid | 99.62% | 99.63% | +0.01% |
| Type I crystal of Compound (1) with citric acid | 99.75% | 99.03% | -0.72% |
| Type IV crystal of Compound (1) with citric acid | 99.91% | 93.49% | -6.42% |
| Type I crystal of Compound (1) with sorbic acid | 99.74% | 99.72% | -0.02% |
| Type I crystal of Compound (1) with phosphoric acid | 99.90% | 99.50% | -0.40% |

As a result, the type III crystal of Compound (1) with succinic acid and the type I crystal of Compound (1) with sorbic acid were found to be extremely stable crystals with almost no increase in the related substances. On the other hand, the type I crystal of Compound (1) with citric acid, the type IV crystal of Compound (1) with citric acid, and the type I crystal of Compound (1) with phosphoric acid were found to be unstable crystals with decreases in purity.

### Test Example 4: Dynamic vapor sorption (DVS) test

Vapor sorption tests were performed using the type III crystal of Compound (1) with succinic acid, the type I crystal of Compound (1) with citric acid, the type IV crystal of Compound (1) with citric acid, the type I crystal of Compound (1) with sorbic acid, and the type I crystal of Compound (1) with phosphoric acid obtained in the examples, reference example, and comparative examples.

Vapor sorption tests were conducted under the same conditions as in Test Example 2.

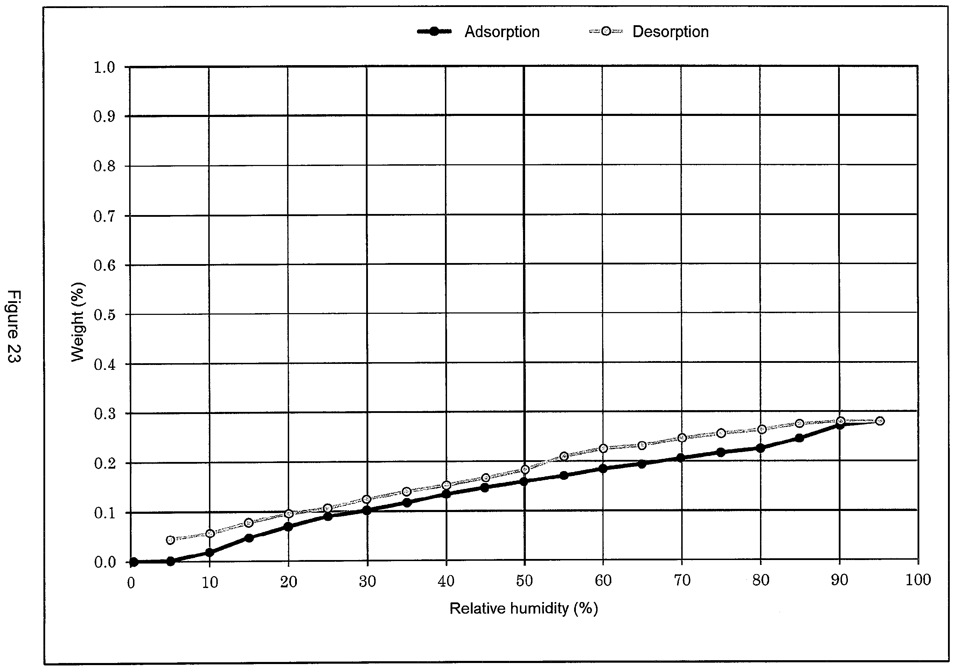

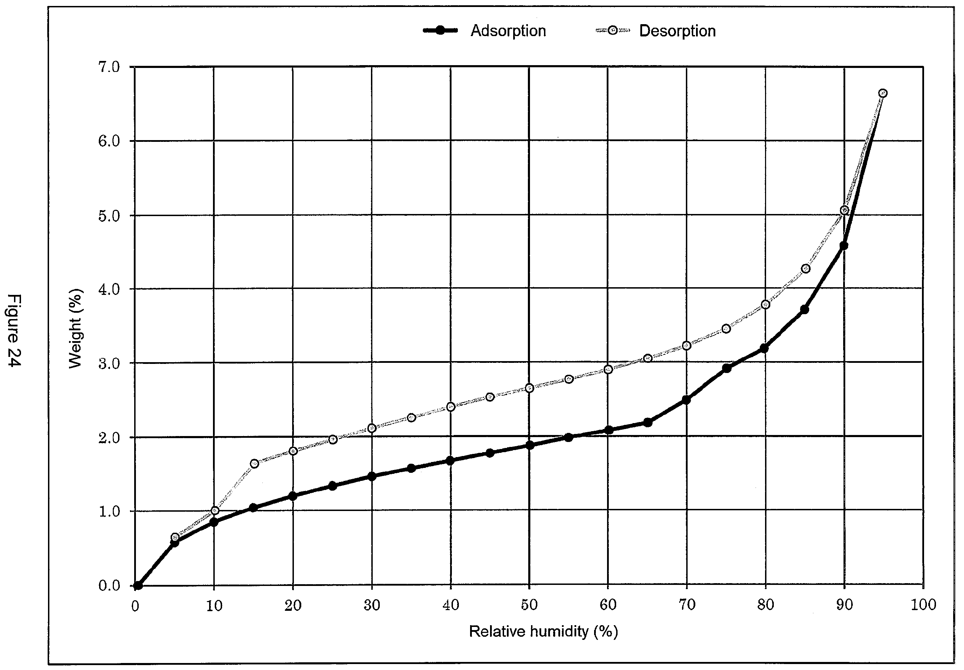

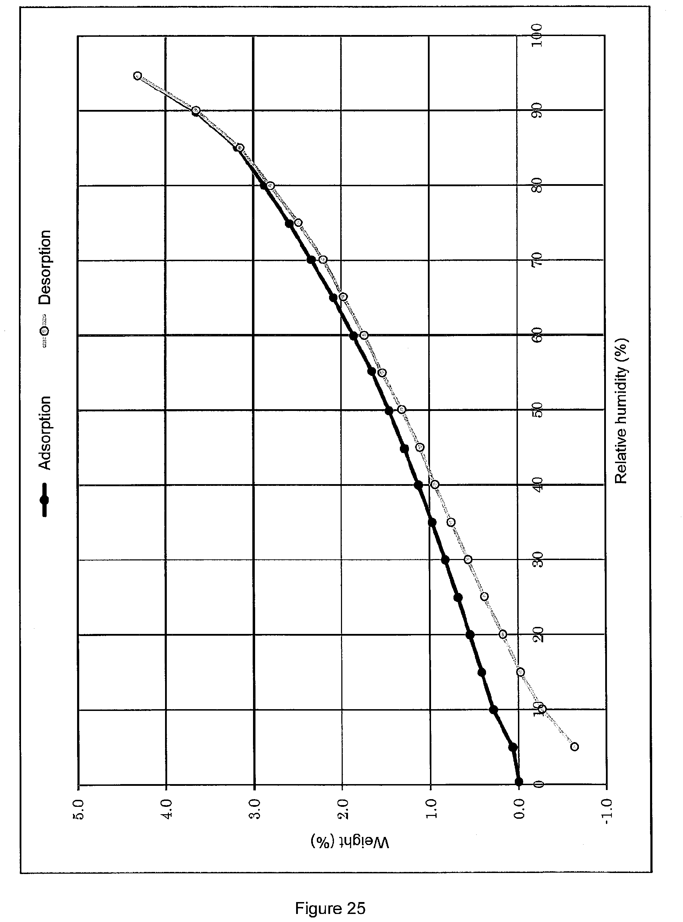

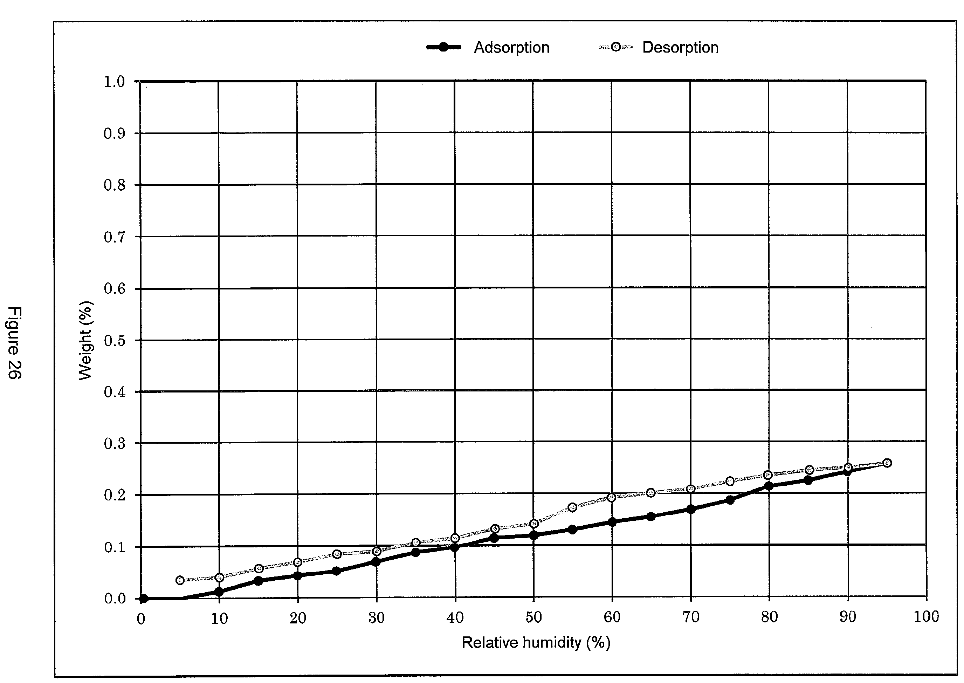

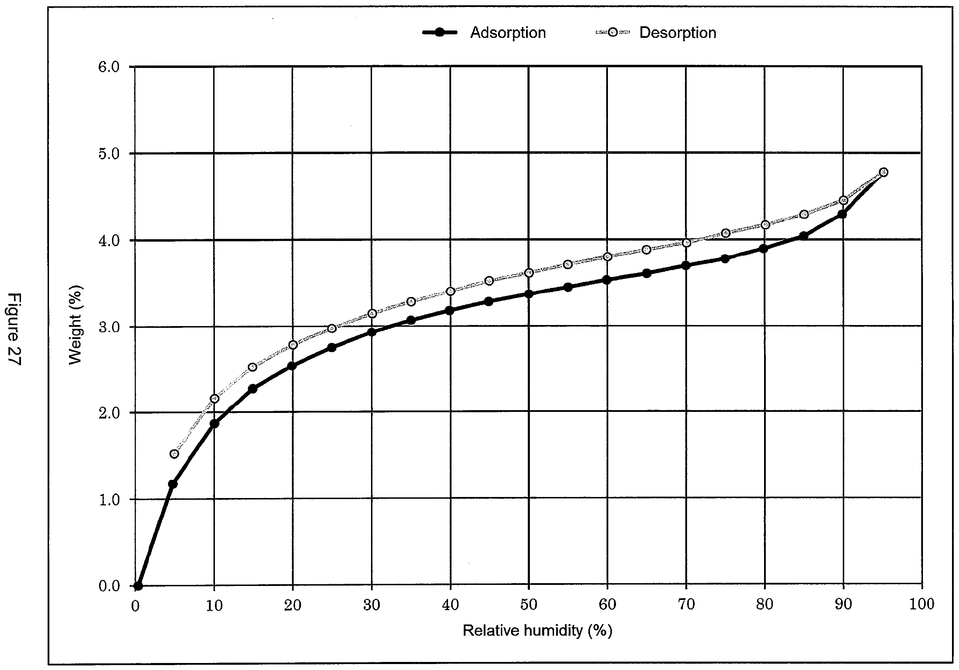

Changes in the weight over the range of the measurement conditions obtained in these tests are shown in Figures 23 through 27.

As shown in the figures, the type III crystal of Compound (1) with succinic acid and the type I crystal of Compound (1) with sorbic acid showed weight increases of 0.28% and 0.26%, respectively, under 95% relative humidity in the vapor sorption test, and these weight increases of less than 1% indicate that they were hardly hygroscopic. On the other hand, as shown in the figures, the type I crystal of Compound (1) with citric acid, the type IV crystal of Compound (1) with citric acid, and the type I crystal of Compound (1) with phosphoric acid each showed a mass increase of 1% or more under 95% relative humidity in the vapor sorption test, indicating that they were highly hygroscopic.

The above results showed that the type III crystal of Compound (1) with succinic acid and the type I crystal of Compound (1) with sorbic acid had lower hygroscopicity than the type I crystal of Compound (1) with citric acid, the type IV crystal of Compound (1) with citric acid, and the type I crystal of Compound (1) with phosphoric acid, and were superior in terms of industrial manufacturing of a pharmaceutical product of stable quality among the candidate compounds for the development of the pharmaceutical product. The type III crystal of Compound (1) with succinic acid and the type I crystal of Compound (1) with sorbic acid were confirmed to have excellent properties as pharmaceutical products or active pharmaceutical ingredients.

### Test Example 5: Plasma concentration determination test

The Compound (1) in free form (type II crystal), the type III crystal of Compound (1) with one equivalent of succinic acid, and the type I crystal of Compound (1) with one equivalent of sorbic acid obtained in the examples were tested for plasma concentrations in male beagle dogs after oral administration under hypochlorhydria conditions.

### (Selection of beagle dogs)

In order to select three male beagle dogs for the test, plasma concentrations were determined in five male beagle dogs under hypochlorhydria conditions after administration in a solution form and administration in a capsule-filled form. Three dogs with larger differences in AUCₗₐₛₜ (area under the plasma concentration-time curve from 0 hours after administration to the point at which the final concentration can be calculated by the linear trapezoidal method before Tₘₐₓ and by the log-trapezoidal method after Tₘₐₓ) and Cₘₐₓ (maximum plasma concentration) between the administration in the solution form and the administration in the capsule-filled form were selected. Subsequently, tests were conducted to determine plasma concentrations in them after oral administration of the Compound (1) in free form (type II crystal), the type III crystal of Compound (1) with one equivalent of succinic acid, and the type I crystal of Compound (1) with one equivalent of sorbic acid in capsule-filled forms. The following procedures were employed for the preparation of a liquid dosage, capsule filling, and animal experiments.

### (Methods for preparation of liquid dosage, capsule filling, and animal experiments)

Compound (1) in free form was dissolved in a 20% HP-β-CD solution containing 0.1N hydrochloric acid to prepare a solution of 20 mg/5 mL. Compound (1) in free form (type II crystal), the type III crystal of Compound (1) with one equivalent of succinic acid, and the type I crystal of Compound (1) with one equivalent of sorbic acid were each filled into a gelatin capsule at 20 mg/kg in terms of the molecular weight of Compound (1) (free form).

The male beagle dogs selected for the study were fasted starting between 11:00 a.m. to 12:00 p.m. on the day before Compound (1) was administered and fed after blood was collected 8 hours after the administration of the compound. The dogs were not allowed to drink water from 30 minutes before to 2 hours after the administration of the compound, but were allowed to drink freely at other time.

Intravenous administration of atropine (atropine sulfate injection 0.5 mg "Fuso") at a dose of 0.02 mg/kg was followed by intramuscular administration of pentagastrin at a dose of 0.01 mg/kg 30 minutes prior to the administration of the compound. Thirty minutes after the administration of atropine, the dogs were orally force-fed a capsule filled with the compound, encouraged to self-swallow the capsule with 5 mL of distilled water, and further orally force-fed 50 mL of distilled water using a catheter. Alternatively, the compound dissolved in liquid dosage was administered orally via a catheter at a dose of 20 mg/5 mL/kg, and the catheter was immediately rinsed with 10 mL of distilled water.

Pentagastrin was again intramuscularly administered at 0.01 mg/kg 15 minutes and 1 hour after the administration of the compound.

Before and after (0.25, 0.5, 1, 2, 4, 6, 8, and 24 hours after) the administration, the person in charge of collecting blood held the dog's forelimb to distend the blood vessel using a clothespin or the like. Then, the blood collection site was disinfected with alcohol cotton, and about 0.2 mL of blood was collected using a heparinized syringe and needle. After the blood was drawn, the puncture site was pressed with a cotton pad to adequately stop bleeding. The collected blood samples were ice-cooled, and plasma was isolated by centrifugation as soon as possible. The isolated plasma was immediately frozen on dry ice and then stored frozen in a freezer set at -80°C.

AUCₗₐₛₜ, Cₘₐₓ, and Tₘₐₓ (time to reach maximum blood concentration) were calculated using Phoenix WinNonlin (v7.0.0, Certara USA, Inc.) from the concentration of Compound (1) in each plasma sample measured by MRM method and quantified from the calibration curve using LC/MS/MS.

The results are shown in Table 8. The type II crystal of Compound (1) in free form, the type III crystal of Compound (1) with succinic acid, and the type I crystal of Compound (1) with sorbic acid were assumed to be crystalline forms that can exert medicinal effects *in vivo* as well. These results confirmed that all of the crystalline forms were orally administrable.

**Table 8**

| | Type II crystal of Compound (1) in free form | Type III crystal of Compound (1) with succinic acid | Type I crystal of Compound (1) with sorbic acid |
|---|---|---|---|
| AUCₗₐₛₜ (µM·hr) | 13.0 ± 4.0 | 25.1 ± 8.4 | 18.1 ± 13.1 |
| Cₘₐₓ (µM) | 1.26 ± 0.09 | 2.24 ± 0.75 | 1.68 ± 0.88 |
| tₘₐₓ (hr) | 4.0 ± 0.0 | 5.3 ± 1.2 | 6.0 ± 2.0 |

All references and publications cited herein are incorporated herein by reference in their entirety regardless of their purposes. This specification also includes the disclosure of the claims, description, and drawings of Japanese patent application No. 2022-071154 (filed on April 22, 2022), which is the basis for the priority claim of this application.

Some embodiments of the present invention have been described but these embodiments are presented as examples and are not intended to limit the scope of the invention. These novel embodiments can be implemented in various other forms, and various omissions, replacements, and modifications can be made without departing from the gist of the invention. These embodiments and variations thereof are encompassed by the scope and gist of the invention as well as by the scope of the invention stated in the claims and equivalents thereof.

## Claims

1. A type II crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide,
wherein the crystal has peaks in a powder X-ray diffraction spectrum at diffraction angles (2θ ± 0.2°) of:
(i) one or more selected from the group consisting of 15.6°, 20.1°, 24.7°, and 28.9°; and
(ii) two or more selected from the group consisting of 7.8°, 10.6°, 14.0°, 14.8°, 17.2°, 21.6°, 22.2°, and 25.8°.

2. The crystal according to claim 1, wherein the crystal has peaks in a powder X-ray diffraction spectrum at a total of five or more diffraction angles (2θ ± 0.2°) selected from groups (i) and (ii) above.

3. The crystal according to claim 1 or 2, wherein the crystal has peaks in a powder X-ray diffraction spectrum at a total of seven or more diffraction angles (20 ± 0.2°) selected from groups (i) and (ii) above.

4. The crystal according to any one of claims 1 to 3, wherein the crystal has a powder X-ray diffraction spectrum substantially identical to the powder X-ray diffraction spectrum shown in Figure 3.

5. The crystal according to any one of claims 1 to 4, wherein an exothermic peak determined by simultaneous thermogravimetric-differential thermal analysis is around 236°C.

6. The crystal according to any one of claims 1 to 5, wherein the crystal has a crystal purity of 95% by weight or more.

7. The crystal according to any one of claims 1 to 6, wherein the crystal has a chemical purity of 95% or more.

8. A method for producing the type II crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide according to any one of claims 1 to 7, the method comprising stirring N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide in a single solvent of a lower alcohol with 3 or more carbon atoms, tetrahydrofuran, acetone, or ethyl acetate, or in a mixed solvent comprising two or more of these solvents.

9. A type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with succinic acid, wherein the crystal has peaks in a powder X-ray diffraction spectrum at three or more diffraction angles (2θ ± 0.2°) selected from the group consisting of 10.4°, 13.3°, 14.6°, 15.7°, 17.2°, 18.3°, 19.7°, 20.8°, 22.3°, and 27.5°.

10. The crystal according to claim 9, wherein the crystal has peaks in a powder X-ray diffraction spectrum at five or more diffraction angles (2θ ± 0.2°) selected from the group consisting of 10.4°, 13.3°, 14.6°, 15.7°, 17.2°, 18.3°, 19.7°, 20.8°, 22.3°, and 27.5°.

11. The crystal according to claim 10 or 11, wherein the crystal has peaks in a powder X-ray diffraction spectrum at seven or more diffraction angles (2θ ± 0.2°) selected from the group consisting of 10.4°, 13.3°, 14.6°, 15.7°, 17.2°, 18.3°, 19.7°, 20.8°, 22.3°, and 27.5°.

12. The crystal according to any one of claims 9 to 11, wherein the crystal has a powder X-ray diffraction spectrum substantially identical to the powder X-ray diffraction spectrum shown in Figure 13.

13. The crystal according to any one of claims 9 to 12, wherein an endothermic peak determined by simultaneous thermogravimetric-differential thermal analysis is around 202°C.

14. The crystal according to any one of claims 9 to 13, wherein the crystal has a crystal purity of 95% by weight or more.

15. The crystal according to any one of claims 9 to 14, wherein the crystal has a chemical purity of 95% or more.

16. A method for producing the type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with succinic acid according to any one of claims 9 to 15, the method comprising stirring N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide and succinic acid at an equivalent ratio of 1:1-10 in a single solvent selected from acetonitrile, a lower alcohol with 2 or more carbon atoms, methyl ethyl ketone, tetrahydrofuran, or 2-methyltetrahydrofuran, or in a mixed solvent comprising two or more of these solvents.

17. A type I crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with sorbic acid, wherein the crystal has peaks in a powder X-ray diffraction spectrum at three or more diffraction angles (2θ ± 0.2°) selected from the group consisting of 12.6°, 14.7°, 15.4°, 15.9°, 17.1°, 18.9°, 22.2°, 24.1°, 25.8°, and 27.4°.

18. The crystal according to claim 17, wherein the crystal has peaks in a powder X-ray diffraction spectrum at five or more diffraction angles (2θ ± 0.2°) selected from the group consisting of 12.6°, 14.7°, 15.4°, 15.9°, 17.1°, 18.9°, 22.2°, 24.1°, 25.8°, and 27.4°.

19. The crystal according to claim 17 or 18, wherein the crystal has peaks in a powder X-ray diffraction spectrum at seven or more diffraction angles (2θ ± 0.2°) selected from the group consisting of 12.6°, 14.7°, 15.4°, 15.9°, 17.1°, 18.9°, 22.2°, 24.1°, 25.8°, and 27.4°.

20. The crystal according to any one of claims 17 to 19, wherein the crystal has a powder X-ray diffraction spectrum substantially identical to the powder X-ray diffraction spectrum shown in Figure 15.

21. The crystal according to any one of claims 17 to 20, wherein an endothermic peak determined by simultaneous thermogravimetric-differential thermal analysis is around 173°C.

22. The crystal according to any one of claims 17 to 21, wherein the crystal has a crystal purity of 95% by weight or more.

23. The crystal according to any one of claims 17 to 22, wherein the crystal has a chemical purity of 95% or more.

24. A method for producing the type I crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide with sorbic acid according to any one of claims 17 to 23, the method comprising stirring N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide and sorbic acid at an equivalent ratio of 1:1-10 in a single solvent selected from acetonitrile, a lower alcohol, tetrahydrofuran, acetone, methyl ethyl ketone, water, or ethyl acetate, or in a mixed solvent comprising two or more of these solvents.

25. A type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide, wherein the crystal has peaks in a powder X-ray diffraction spectrum at diffraction angles (2θ ± 0.2°) of:
(i) one or more selected from the group consisting of 8.8°, 11.6°, and 17.9°; and
(ii) two or more selected from the group consisting of 6.6°, 12.3°, 13.8°, 18.6°, 21.3°, and 22.3°.

26. The crystal according to claim 25, wherein the crystal has peaks in a powder X-ray diffraction spectrum at a total of five or more diffraction angles (20 ± 0.2°) selected from groups (i) and (ii) above.

27. The crystal according to claim 25 or 26, wherein the crystal has peaks in a powder X-ray diffraction spectrum at a total of seven or more diffraction angles (2θ ± 0.2°) selected from groups (i) and (ii) above.

28. The crystal according to any one of claims 25 to 27, wherein the crystal has a powder X-ray diffraction spectrum substantially identical to the powder X-ray diffraction spectrum shown in Figure 1.

29. The crystal according to any one of claims 25 to 28, wherein an exothermic peak determined by simultaneous thermogravimetric-differential thermal analysis is around 228°C.

30. The crystal according to any one of claims 25 to 29, wherein the crystal has a crystal purity of 95% by weight or more.

31. The crystal according to any one of claims 25 to 30, wherein the crystal has a chemical purity of 95% or more.

32. A method for producing the type III crystal of N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide according to any one of claims 25 to 31, the method comprising stirring N-(4-(4-amino-6-ethynyl-5-(quinolin-3-yl)-7H-pyrrolo[2,3-d]pyrimidin-7-yl)bicyclo[2.2.1]heptan-1-yl)-5-methylpyrazine-2-carboxamide in a methanol-containing solvent.

33. A pharmaceutical composition comprising the crystal according to any one of claims 1 to 7, 9 to 15, 17 to 23, and 25 to 31.
